# EUROPÄISCHE PATENTSCHRIFT

(11) **EP 0 644 196 B1**
(45) Veröffentlichungstag und Bekanntmachung des Hinweises auf die Patenterteilung: **05.11.1997**
(21) Anmeldenummer: 94810522.6
(22) Anmeldetag: 08.09.1994
(51) Int. Cl.: C07H 19/04, C07H 21/00

(54) **TTTr als Schutzgruppe in der Nukleotidsynthese**
TTTr as a protecting group in nucleotide synthesis
TTTr comme groupe protégéant dans la synthèse des nucléotides

(30) Priorität: 17.09.1993 CH 2813/93
(43) Veröffentlichungstag der Anmeldung: 22.03.1995
(73) Patentinhaber: Novartis AG, 4058 Basel (CH)
(72) Erfinder: Brill, Wolfgang K. D., Dr., D-79650 Schopfheim (DE)

(56) Entgegenhaltungen:
- TETRAHEDRON, (INCL. TETRAHEDRON REPORTS), Bd.48, Nr.12, 1992, OXFORD GB Seiten 2223 - 2311 S.L.BEAUCAGE ET AL. 'Advances in the Synthesis of Oligonucleotides by the Phosphoramidite Approach.'
- JOURNAL OF ORGANIC CHEMISTRY., Bd.58, Nr.14, 1993, EASTON US Seiten 3748 - 3756 H.W.GIBSON ET AL. 'New Triarylmethyl Derivatives: "Blocking Groups" for Rotaxanes and Polyrotaxanes.'

## Beschreibung

Die vorliegende Erfindung betrifft Nukleoside, Nukleotide und Oligonukleotide, deren Grundgerüst eine primäre, durch Tris-4,4',4''-tert.butyltrityl geschützte Hydroxylgruppe enthält, Verfahren zur Herstellung dieser Nukleoside und Nukleotide, ein Verfahren zur Herstellung von Oligonukleotiden sowie die Verwendung von derartig geschützten Nukleosiden, Nukleotiden und Oligonukleotiden.

Vor der Synthese von Oligonukleotiden, die wegen ihrer Fähigkeit zur Wechselwirkung mit Nukleinsäuren (u. a. "Antisense"-Oligonukleotide) und der damit verbundenen biologischen Aktivität breites Interesse gefunden haben, werden die verwendeten Nukleoside und Nukleosidanaloga an ihren 5'-Positionen mit Schutzgruppen versehen. Es sind dies beispielsweise Phenylxanthen-9-yl- (Pixyl-) und Tritylschutzgruppen [Beaucage, S.L., Iyer, R., Tetrahedron 48:2223-2311 (1992)]. Sowohl bei der Pixyl- als auch bei der Di-p-anisylphenylmethyl- (DMTr-) und der p-Anisyldiphenylmethyl- (MMTr-) Gruppe führt die geringe Regioselektivität bereits bei der Einführung zu Ausbeuteverlusten. Ein weiterer Nachteil dieser Tritylgruppen mit langen O-Alkylketten ist, dass zwar die chromatographische Reinigung der aus ihnen hervorgehenden Oligonukleotide erleichtert ist, die durch sie geschützten Nukleosidmonomeren jedoch nur eine geringe Tendenz haben, Feststoffe oder kristalline Körper zu bilden, wodurch ihre technische Anwendung stark eingeschränkt ist [Takenaka, S., Dohtsu, K., Takagi, M., Anal. Sci. 8:3-7 (1992)].

Die Tris-4,4',4''-methoxytritylgruppe ist für die Oligonukleotidsynthese zu labil und wird unter den Kopplungsbedingungen des Phosphittriester-Verfahrens abgespalten [Beaucage, S.L., Iyer, R., Tetrahedron 48:2233 (1992)]. Die 4,4',4''-Tris-(benzoyloxy)trityl- (TBTr-) Gruppe und die 4,4',4''-Tris-(4,5-dichlorphthalimido)trityl- (CPTr-) Gruppe sind aufgrund ihrer für Schutzgruppen komplizierten Herstellungs- und Abspaltungsbedingungen zu umständlich für eine generelle Anwendung und besonders bei der automatisierten Synthese [Beaucage, S.L., Iyer, R., Tetrahedron 48:2235 (1992)]. Andere Tritylgruppen [Beaucage, S.L., Iyer, R., Tetrahedron 48:2236 (1992)] lassen die Derivatisierung der Nukleosidmonomeren nur in mässigen Ausbeuten zu. Eine Kristallisation der Derivate ist in der Regel unmöglich.

Es wurde nun überraschend gefunden, dass Nukleoside und Nukleosidanaloge dann als leicht zu reinigende amorphe oder kristalline Feststoffe anfallen, wenn die 5'-Hydroxygruppe mit Tris-4,4',4''-tert.butylphenylmethyl (= Tris-4,4',4''-tert.butyltrityl oder TTTr) geschützt ist. Es wurde ferner überraschend gefunden, dass sich Oligonukleotide, die an ihrem 5'-Terminus eine TTTr-Gruppe tragen, wesentlich leichter reinigen lassen, besonders durch chromatographische Methoden wie die reversed phase HPLC.

Ein Gegenstand der vorliegenden Erfindung sind Nukleoside, Nukleosidanaloge, Nukleotide, Ninkleotidanaloge oder Oligonukleotide aus mindestens zwei solcher gleichen oder verschiedenen Nukleotide und/oder Nukleotidanalogen, deren Grundgerüst einen gegebenenfalls substituierten Rest einer Nukleinbase B und jeweils eine primäre, geschützte Hydroxylgruppe enthält, wobei die Schutzgruppe TTTr ist.

Die TTTr-Gruppe selbst ist bekannt. H.W. Gibson et al., J. Org. Chem. 58:3748-3756 (1993), beschreiben die Verwendung einer TTTr-Gruppe als Synthesebaustein zur Herstellung von Rotaxanen.

Marvel et al. [Marvel, C.S., Kaplan, J.F., Himel, C.M., J. Am. Chem. Soc. 63:1892-1896 (1941)] offenbaren TTTrCl, TTTrOH und Peroxide der TTTr-Gruppe im Zusammenhang mit dem Dissoziationsverhalten von Alkylsubstituierten Hexaarylethanen. Ashton et al. [Ashton, P.R., Philp, D., Spencer, N., Stoddart, J.F., J. Chem. Soc., Chem. Commun. 1124-1128 (1992)] beschreiben die TTTr-Gruppe als Sperrgruppe beim Aufbau von Vorrichtungen im Nanometer-Bereich, insbesondere beim Aufbau von sogenannten Rotaxanen und Pseudorotaxanen.

Als neue Schutzgruppe in der Nukleinsäurechemie zeichnet sich die TTTr-Schutzgruppe durch ihre überraschend leichte Einführbarkeit aus, die mit grösserer Regioselektivität als bei den gebräuchlichen DMTr- und Pixyl-Gruppen erfolgt. Die hierbei entstehenden erfindungsgemässen Nukleoside und Nukleosidanaloga lassen sich oft ohne Chromatographie aus den Reaktionsmischungen isolieren, wodurch ihre Verwendung bei der grosstechnischen Darstellung von Nukleosidbausteinen für die DNA-Synthese (automatisierte Synthesen) sehr vorteilhaft ist. Ausserdem können die erfindungsgemässen Oligonukleotide leichter mittels bekannter Verfahren, vorteilhafterweise durch reversed phase HPLC, gereinigt werden als jene Oligonukleotide, welche die herkömmlichen Schutzgruppen tragen.

Die TTTr-Gruppe kann gleichzeitig mit den allgemein bekannten Schutzgruppen der Oligonukleotidchemie [Beaucage, S.L., Iyer, R., Tetrahedron 48:Tabellen 1 bis 3 (1992)], vorteilhafterweise mit einer Schutzgruppe freier Aminogruppen in den Nukleinbasen, beispielsweise mit einer gegebenenfalls substituierten Cycloalkylcarbonylgruppe mit 3 bis 12, vorzugsweise 4 bis 8, besonders bevorzugt mit 5 oder 6 Ringkohlenstoffatomen, insbesondere mit der Cyclohexancarboxylgruppe (CC), oder einer Amidinschutzgruppe, beispielsweise der Dimethylaminomethylidengruppe verwendet werden. Die erfindungsgemässen Nukleoside und Nukleosidanaloga eignen sich als Bausteine für die Oligonukleotidsynthese mittels Festphaseverfahren und selbst nach einem Verfahren in Lösung.

Im Rahmen der vorliegenden Erfindung geeignete Nukleoside, Nukleosidanaloge, Nukleotide, Nukleotidanaloge oder Oligonukleotide mit einer der Knüpfung der Nukleotidbindung dienenden vorzugsweise sekundären OH-Gruppe sind in grosser Vielzahl bekannt und in der Fachliteratur beschrieben, beispielsweise in Townsend, L.B. (Hrsg.), Chemistry of Nucleosides and Nucleotides 1, Plenum Press, New York (1988) oder nach bekannten Verfahren herstellbar. Es kann sich bei ihnen im allgemeinen um ein gegebenenfalls mit -O- oder -S- unterbrochenes offenkettiges C-Gerüst oder carbacyclisches oder O- bzw. S-heterocyclisches Grundgerüst mit einer Nukleinbase B handeln. Bei den Nukleosiden kann es sich um natürliche oder synthetische Nukleoside handeln.

Das offenkettige Kohlenstoff-Gerüst kann zum Beispiel 3 bis 12, bevorzugt 3 bis 6 Kohlenstoff-Atome enthalten. Bei den carbacyclischen und heterocyclischen Grundgerüsten kann es sich zum Beispiel um monocyclische Ringsysteme mit 3 bis 12, bevorzugt 3 bis 8 und besonders bevorzugt 4 oder 5 Ringkohlenstoffatomen handeln. Es kann sich auch um bicyclische bis tetracyclische Systeme mit zum Beispiel 5 bis 16, bevorzugt 8 bis 16 Kohlenstoff-Atomen handeln. Die Grundgerüste können weitere Substituenten enthalten, zum Beispiel geschützte OH-Gruppen.

In einer bevorzugten Ausführungsform handelt es sich bei den Nukleosiden um 5-gliedrige Carbacyclen oder Furane.

Wenn die Nukleinbase B einen Purinrest oder ein Analoges davon darstellt, so kann es sich um Reste der Formeln I, Ia, Ib, Ic, Id oder Ie handeln, 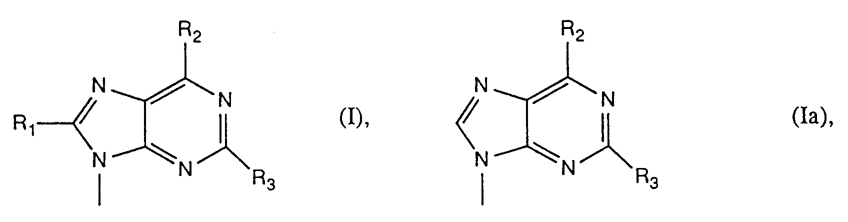 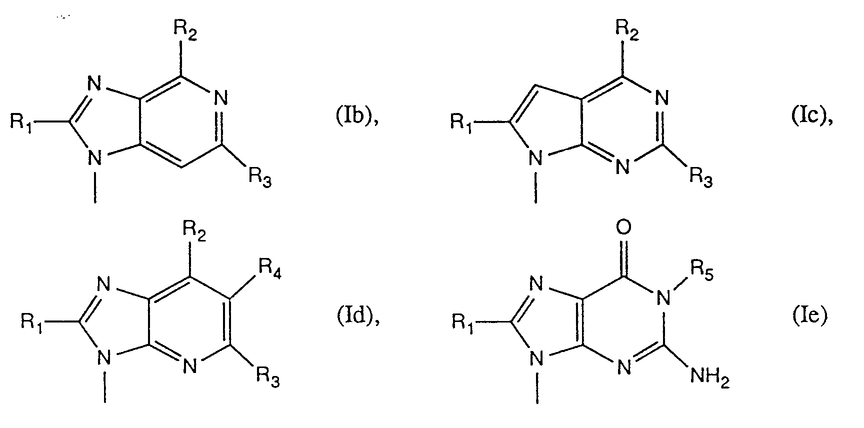 worin R₁ für H, Cl, Br, NH₂ oder OH steht, und R₂, R₃ und R₄ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₅ H oder C₁-C₄-Alkyl darstellt.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminoalkylreste sind Methyl, Ethyl, n-und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel R₆R₇N handeln, worin R₆ für H steht oder unabhängig die Bedeutung von R₇ hat, und R₇ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₆ und R₇ zusammen Tetra- oder Pentamethylen, 3-Oxa-1,5-pentylen, -CH₂-NR₈-CH₂CH₂- oder -CH₂CH₂-NR₈-CH₂CH₂- darstellen, worin R₈ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder -aminopropyl oder -aminobutyl. Beispiele für Hydroxyalkyl sind Hydroxymethyl, 1-Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder - 4-yl, Pent-3- oder 4-en-1-yl oder -2-yl, Hex-3- oder -4- oder -5-en-1-yl oder -2-yl. Beispiele für Alkyl-und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. R₈ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino und Benzoylamino.

In einer bevorzugten Ausführungsform stellt R₁ Wasserstoff dar. In einer anderen bevorzugten Ausführungsform stellt R₄ Wasserstoff dar. In einer weiteren bevorzugten Ausführungsform bedeuten R₂ und R₃ unabhängig voneinander H, F, Cl, Br, OH, SH, NH₂, NHOH, NHNH₂, Methylamino, Dimethylamino, Benzoylamino, Methoxy, Ethoxy und Methylthio.

Einige Beispiele für Analoge der Purinreihe sind neben Purin Adenin, N-Methyladenin, N-Benzyladenin, 2-Methyladenin, 2-Methylthioadenin, 2-Aminoadenin, 3-Carbaadenin, 7-Carbaadenin, 1-Carbaadenin, 6-Hydroxypurin, 2-Amino-6-chlorpurin, 2-Amino-6-methylthiopurin, 2-Amino-6-hydroxypurin, 3-Carba-6-chlorpurin, Guanin, 2-Methylguanin. Besonders bevorzugt sind Adenin, 2-Aminoadenin und Guanin.

Wenn die Nukleinbase B einen analogen Pyrimidinrest darstellt, so handelt es sich um Reste der Formeln II, IIa und IIb, 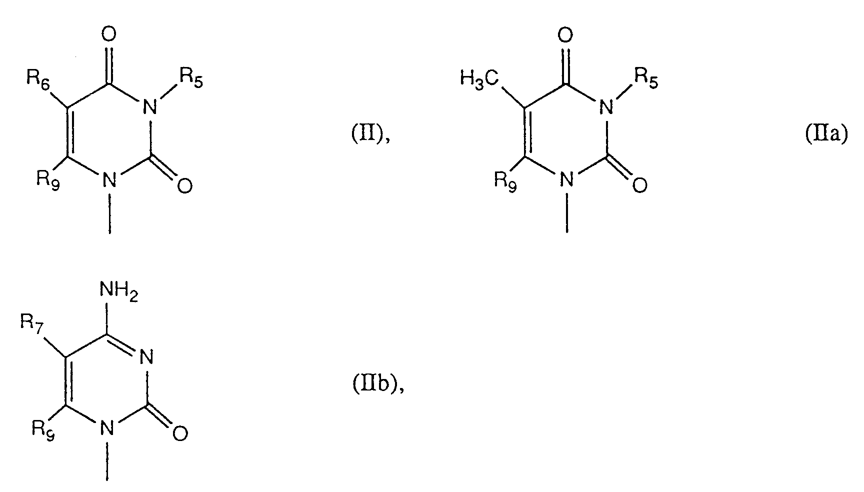 worin R₅ H oder C₁-C₄-Alkyl bedeutet, und R₆, R₇ und R₉ unabhängig voneinander die zuvor für R₂ angegebene Bedeutung haben, einschliesslich der Bevorzugungen, und die Wasserstoffatome der NH₂-Gruppe in Formel IIb mit C₁-C₆-Alkyl oder Benzoyl substituiert sein können, sowie die Dihydroderivate der Reste der Formeln II, IIa und IIb. Bevorzugt stellt R₆ H, C₁-C₆-Alkyl oder Hydroxyalkyl, F, Cl, Br, NH₂, Benzoylamino, Mono- oder Di-C₁-C₆-alkylamino dar und R₇ stellt bevorzugt H, C₁-C₆-Alkyl oder -Alkoxy oder -Hydroxyalkyl, F, Cl, Br, NH₂, Benzoylamino, Mono- oder Di-C₁-C₆-alkylamino dar.

R₅ steht bevorzugt für H oder Methyl. R₆ bedeutet bevorzugt H, F, Cl, Br, NH₂, NHCH₃, N(CH₃)₂ oder C₁-C₄-Alkyl. R₇ stellt bevorzugt H, C₁-C₄-Alkyl, besonders Methyl, oder NH₂, NHCH₃ oder (CH₃)₂N dar.

Einige Beispiele für Pyrimidinanaloge sind Uracil, Thymin, Cytosin, 5-Fluoruracil, 5-Chloruracil, 5-Bromuracil, Dihydrouracil, Pseudouracil, 1-Methylpseudouracil, 5-Methyluracil, 3-Methylcytosin und 5-Methylcytosin.

Im Rahmen der vorliegenden Erfindung sind als Schutzgruppen für Nukleinbasen allgemein in der Fachwelt bekannte Schutzgruppen gemeint. Beispiele für solche Schutzgruppen sind: C₁-C₈-Alkyl; mono- oder bicyclisches C₇-C₁₂-Aralkyl; mono- oder bicyclisches C₇-C₁₂-Aralkoxy; mono- oder bicyclisches C₇-C₁₂-Haloaralkyl; Diphenylmethyl; Diphenylmethyl, substituiert mit 1 bis 4 Methyl oder Methoxy; Triphenylmethyl; Triphenylmethyl, substituiert mit 1 bis 6 Methyl oder Methoxy, oder 1 bis 3 tert.-Butyl; Xanthenyl, substituiert mit Phenyl oder Naphthyl; -Si(R₁₀)(R₁₁)(R₁₂), worin (R₁₀)(R₁₁) und (R₁₂) unabhängig voneinander C₁-C₂₀-Alkyl, Benzyl oder Phenyl bedeuten; R-C(O)-, worin R C₁-C₆-Alkyl, Benzyl, Benzyl, substituiert mit Methyl, Methoxy oder Halogen, C₁-C₁₂-Alkoxy, C₁-C₁₂-Alkoxy, substituiert mit Fluoren, Phenyloxy, Phenyloxy, substituiert mit Methyl, Methoxy oder Halogen, Benzyloxy oder Benzyloxy, substituiert mit Methyl, Methoxy oder Halogen bedeutet; R₁₃-SO₂-, worin R₁₃ C₁-C₁₂-Alkyl, C₅-C₆-Cycloalkyl, Phenyl, Phenyl, substituiert mit C₁-C₁₂-Alkyl oder Halogen, Benzyl oder Benzyl, substituiert mit C₁-C₁₂-Alkyl oder Halogen bedeutet; C₁-C₁₂-Alkoxyacetyl oder Phenoxyacetyl, das unsubstituiert oder mit linearem oder verzweigten C₁-C₆-Alkyl, C₁-C₆-Halogenalkyl, Halogen, C₁-C₆-Alkoxy, C₁-C₆-Alkylthio, Nitro oder Cyano ein- oder mehrfach substituiert sein kann; Cycloalkylcarbonyl mit 3 bis 12 Ringkohlenstoffatomen; mit C₁-C₆-Alkyl oder C₁-C₆-Alkoxy substituiertes Cycloalkylcarbonyl mit 3 bis 12 Ringkohlenstoffatomen; oder Amidinschutzgruppen, beispielsweise der Dimethylaminomethylidengruppe.

Wie bereits erwähnt sind die Cycloalkylcarbonylgruppe mit 3 bis 12, vorteilhafterweise mit 4 bis 8, besonders vorteilhaft mit 5 oder 6 Ringkohlenstoffatomen, insbesondere die Cyclohexancarbonylgruppe, und die Dimethylaminomethylidengruppe bevorzugt.

Beispiele für C₁-C₈-Alkyl sind Methyl, Ethyl, n- und i-Propyl, n-, i- und t-Butyl; für ein monocyclisches C₇-C₁₂-Aralkyl sind Benzyl, Methylbenzyl, Dimethylbenzyl; für ein mono- oder bicyclisches C₇-C₁₂-Aralkoxy sind Methoxybenzyl, Dimethoxybenzyl; für ein mono- oder bicyclisches C₇-C₁₂-Haloalkyl ist Brombenzyl; für ein substituiertes Diphenylmethyl sind Di(methylphenyl)methyl, Di(dimethylphenyl)methyl, Di(methoxyphenyl)methyl, Di(dimethoxyphenyl)methyl; für ein substituiertes Triphenylmethyl sind Tri(methylphenyl)methyl, Tri(dimethylphenyl)methyl, Tri(methoxyphenyl)methyl, Tri(dimethoxyphenyl)methyl, Monomethoxytrityl, Dimethoxytrityl und tris-p-tert.-Butylphenylmethyl; für Silylgruppen sind Triphenylsilyl, Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; für die Gruppe R-C(O)- sind Acetyl, Trifluoracetyl, Propanoyl, Butanoyl, Pentanoyl, Hexanoyl, Benzoyl, Methylbenzoyl, Methoxybenzoyl, Chlorbenzoyl, Brombenzoyl, Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl, 9-Fluorenylmethyloxycarbonyl, Allyloxycarbonyl. Cinnamoyloxycarbonyl, 2,2,2-Trichlorethoxycarbonyl, Trimethylsilylethoxycarbonyl, Chlorethoxycarbonyl, Bromethoxycarbonyl, Morpholinoethoxycarbonyl; und für die Gruppe R₁₃-SO₂- sind Methyl-, Ethyl-, Propyl-, Butyl-, Phenyl-, Benzyl-, p-Brom-, p-Methoxy- und p-Methylphenylsulfonyl; und für Alkoxyacetyl und Phenoxyacetyl Methoxyacetyl, Ethoxyacetyl, Phenoxyacetyl, (p-Methylphenoxy)acetyl, (p-Tert.butylphenoxy)acetyl.

Im Rahmen der vorliegenden Erfindung besonders geeignete Nukleoside oder Nukleotide haben eine der nachfolgenden Formeln IIIa, IIIb, IIIc oder IIId 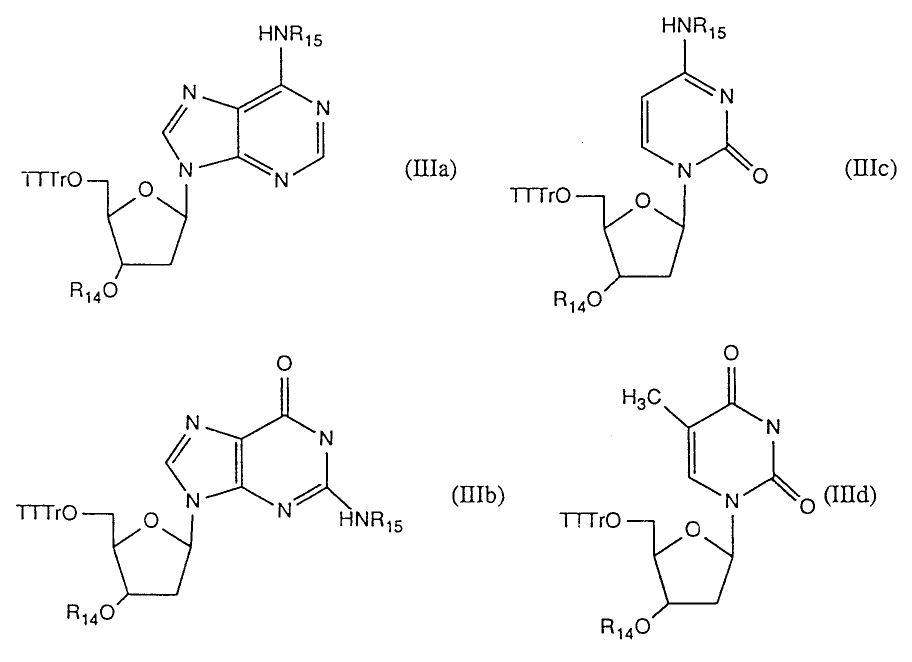 worin R₁₄ Wasserstoff oder einen Nukleotid-Brückengruppe bildenden Rest und R₁₅ Wasserstoff oder Cyclohexylcarbonyl bedeuten.

Im Rahmen der vorliegenden Erfindung besonders geeignete Nukleotide sind solche, bei denen R₁₄ in den Formeln IIIa, IIIb, IIIc und IIId einen phosphorhaltigen, eine Nukleotid-Brückengruppe bildenden Rest der Formel IVa, IVb oder IVc 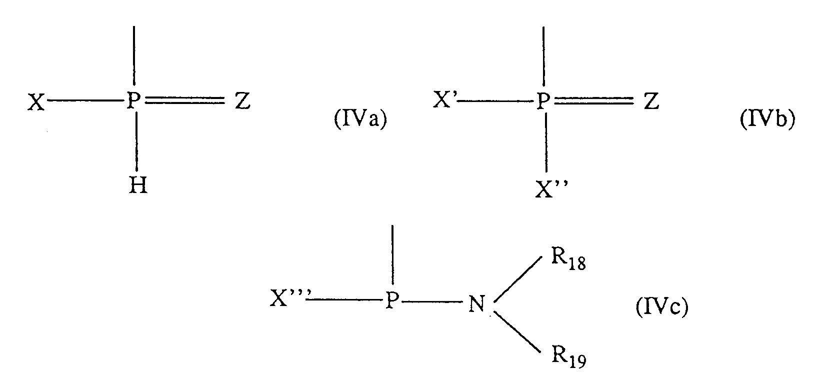 bedeutet, worin
Z Sauerstoff oder Schwefel bedeutet;
X, X' und X'' unabhängig voneinander Sauerstoff oder Schwefel mit negativer Ladung bedeuten, mit Gegenion Li, Na, K, Cs, Tertiär- oder Quartenärammonium; oder
X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten;
X''' C₁-C₁₂-Alkyl, C₆-C₁₂-Aryl, C₇-C₂₀-Aralkyl, C₇-C₂₀-Alkaryl, -OR_{b} oder -SR_{b}, bedeutet; und
R_{b} C₁-C₁₂-Alkyl, C₆-C₁₂-Aryl, C₇-C₂₀-Aralkyl, C₇-C₂₀-Alkaryl bedeutet;
R₁₈ und R₁₉ unabhängig voneinander Wasserstoff, C₁-C₁₂-Alkyl, C₂-C₁₂-Alkenyl, C₆-C₁₂-Aryl, C₇-C₂₀-Aralkyl oder C₇-C₂₀-Alkaryl bedeuten;
   wobei Alkyl, Aryl, Aralkyl und Alkaryl in R₁₈, R₁₉ und R_{b} unsubstituiert oder mit Alkoxy, Alkylthio, Halogen, -CN, -NO₂, Phenyl, Nitrophenyl oder Halogenphenyl substituiert sind.

Unter Tertiär- und Quartenärammonium ist ein Ion der Formel R_{f}R_{g}RₕRᵢN^{⊕} zu verstehen, wobei der Stickstoff des Kations Bestandteil von gesättigten oder ungesättigten mono- bis tricyclischen Ringsystemen sein kann, bei dem R_{f} C₁-C₂₀-, bevorzugt C₁-C₁₂- und besonders bevorzugt C₁-C₆-Alkyl, -Aminoalkyl; Carboxyalkyl oder Carbalkoxyalkyl, wobei die Carbalkoxygruppe 2 bis 8 C-Atome und die Alkylgruppe 1 bis 6, bevorzugt 1 bis 4 C-Atome enthält; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt; und R_{g}, Rₕ und Rᵢ unabhängig voneinander Wasserstoff sind oder die Bedeutung von R_{f} haben, oder R_{f} und R_{g} zusammen Alkyliden, Tetra- oder Pentamethylen, 3-Oxa-1,5-pentylen, -CH₂-NRₑ-CH₂CH₂- oder -CH₂CH₂-NRₑ-CH₂CH₂- darstellen, worin Rₑ für H oder C₁-C₄-Alkyl steht, und Rₕ und Rᵢ unabhängig voneinander die Bedeutung von R_{f} haben. Die Aminogruppe im Aminoalkyl kann mit ein oder zwei C₁-C₄-Alkyl substituiert sein.

Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder - 4-yl, Pent-3- oder -4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl.

Beispiele für R₁₈, R₁₉ und R_{b} als Alkyl sind Methyl, Ethyl und die Isomeren von Propyl, Butyl, Pentyl, Hexyl, Heptyl und Octyl; Beispiele für R₁₈, R₁₉ und R_{b} als Aryl sind Phenyl und Naphthyl; Beispiele für R₁₈ und R₁₉ als Alkenyl sind Allyl und (C₁-C₄-Alkyl)CH=CH-CH₂-; Beispiele für R₁₈, R₁₉ und R_{b} als Aralkyl sind Phenyl-CₙH₂ₙ- mit n gleich einer Zahl von 1 bis 6, besonders Benzyl; Beispiele für R₁₈, R₁₉ und R_{b} als Alkaryl sind Mono-, Di- und Tri(C₁-C₄-alkyl)phenyl. Bevorzugte Substituenten sind Chlor, Brom, Methoxy, -NO₂, -CN, 2,4-Dichlorphenyl und 4-Nitrophenyl. Beispiele für R_{b} sind 2,2,2-Trichlorethyl, 4-Chlorphenyl, 2-Chlorphenyl und 2,4-Dichlorphenyl.

In einer besonders bevorzugten Ausführungsform bedeutet R_{b} β-Cyanoethyl, stellen R₁₈ und R₁₉ Di(i-propyl) dar und steht X''' für O.

Die Nukleotide und Oligonukleotide können kovalent über eine Brückengruppe an ein festes Trägermaterial gebunden sein. Geeignete Trägermaterialien sind zum Beispiel Kieselgele, Controlled Pore Glass, Polystyrol, Polyacrylamid, Polyurethane, Polyolefine, Polyamide, Polyether und veretherte oder acylierte Cellulosederivate. Die Brückengruppe kann je nach Wahl des Trägermaterials zum Beispiel von Dicarbonsäuren, Diurethanen oder Alkoxysilylurethanen abgeleitet sein. Vorbereitete, beladene Trägermaterialien sind kommerziell erhältlich.

Die Einführung der TTTr-Schutzgruppen geschieht analog eines Verfahrens von Gait et al. [Gait, M.J., Matthes, H.W.D., Singh, M., Sproat, B.S., Titmas, R.C., in: Gassen, H.G., Lang, A. (Hrsg.) Chemical and Enzymatic Synthesis of Gene Fragments, Verlag Chemie, Weinheim 1-42 (1982)]. Aminoschutzgruppen, beispielsweise Acylgruppen, werden vorteilhaft vor der TTTr-Gruppe eingeführt. Hierbei können die ungeschützten Nukleoside durch Einwirken von Trialkylchlorsilan, beispielsweise Trimethylchlorsilan, Tert.butyldimethylchlorsilan oder Triisopropylchlorsilan, oder Tetraalkyldisiloxan, beispielsweise 1,1,3,3-Tetraisopropyl-1,3-dichlordisiloxan, in einem geeigneten aprotischen, polaren, basischen Lösungsmittel wie Pyridin, N-Methylmorpholin, Dimethylformamid, Acetonitril oder Tetrahydrofuran bei Raumtemparatur erschöpfend silyliert werden. Anschliessend erfolgt die Umsetzung vorzugsweise mit einem gegebenenfalls substituierten Cycloalkylcarbonsäurechlorid oder mit Dimethylaminomethyliden, wobei nur die Aminofunktionen der Nukleinbase acyliert werden. Es folgt dann die Solvolyse der Silylgruppen durch Einwirkung von geeigneten nukleophilen Agentien, beispielsweise von wässrigem Ammoniak, OH^{⊖} oder F^{⊖}, insbesondere von wässrigem Ammoniumfluorid. Nach Entfernen des Lösungsmittels können die acylierten Nukleoside leicht aufgrund ihres Lösungsverhaltens isoliert werden.

Anschliessend erfolgt die Einführung der TTTr-Gruppe durch Umsetzen des vorzugsweise acylierten Nukleosids mit Tris-4,4',4''-tert.-butyltritylchlorid vorteilhafterweise in Anwesenheit eines sterisch gehinderten tertiären Amins in einem geeigneten aprotischen, polaren, basischen Lösungsmittel wie Pyridin, N-Methylmorpholin, Dimethylformamid, Acetonitril oder Tetrahydrofuran, insbesondere Pyridin. Aufgrund des Löslichkeitsverhaltens der Edukte lassen sich die Produkte leicht abtrennen. Überschüssiges Tritylierungsreagenz kann durch Digerieren mit Petrolether, vorteilhafterweise mit Petrolether (Kp: 40 bis 60°C), entfernt werden.

Die erfindungsgemässen Nukleoside können weiter nach bekannten Verfahren in Phosphoramidit-, H-Phosphonat- oder Triesterderivate überführt werden, welche sich für die Oligonukleotidsynthese nach Festphasenverfahren und nach Verfahren in Lösung eignen.

Ein weiterer Gegenstand der vorliegenden Erfindung ist ein Verfahren zur Herstellung von Oligonukleotiden der Formel V

5'-OH(U)ₘ(V)ₙOH-3' (V)

worin U und V gleiche oder verschiedene, natürliche oder synthetische Nukleosidreste bedeuten und m und n unabhängig voneinander 0 oder eine Zahl von 1 bis 200 darstellen, wobei die Summe aus m und n 2 bis 200 ist, durch
(a) Umsetzung einer Verbindung der Formel VI

   R₂₀-O-(U')_{m'}(V')_{n'}OR₂₁-3' (VI)

   worin R₂₀ eine Schutzgruppe ist und U' und V' die Bedeutungen von U und V haben, m' und n' unabhängig voneinander 0 oder eine Zahl von 1 bis 199 darstellen, wobei die Summe aus m' und n' 2 bis 199 ist, und R₂₁ einen phosphorhaltigen, eine Nukleotid-Brückengruppe bildenden Rest der Formel IVa, IVb oder IVc 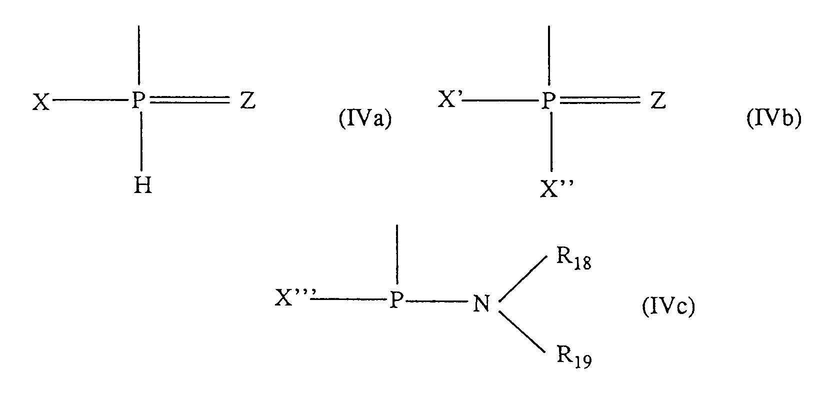 bedeutet, worin
   Z Sauerstoff oder Schwefel bedeutet;
   X, X' und X'' unabhängig voneinander Sauerstoff oder Schwefel mit negativer Ladung bedeuten, mit Gegenion Li, Na, K, Cs, Tertiär- oder Quartenärammonium; oder
   X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten;
   X''' C₁-C₁₂-Alkyl, C₆-C₁₂-Aryl, C₇-C₂₀-Aralkyl, C₇-C₂₀-Alkaryl, -OR_{b} oder -SR_{b}, bedeutet; und
   R_{b} C₁-C₁₂-Alkyl, C₆-C₁₂-Aryl, C₇-C₂₀-Aralkyl, C₇-C₂₀-Alkaryl bedeutet;
   R₁₈ und R₁₉ unabhängig voneinander Wasserstoff, C₁-C₁₂-Alkyl, C₂-C₁₂-Alkenyl, C₆-C₁₂-Aryl, C₇-C₂₀-Aralkyl oder C₇-C₂₀-Alkaryl bedeuten;
      wobei Alkyl, Aryl, Aralkyl und Alkaryl in R₁₈, R₁₉ und R_{b} unsubstituiert oder mit Alkoxy, Alkylthio, Halogen, -CN, -NO₂, Phenyl, Nitrophenyl oder Halogenphenyl substituiert sind,
      mit einer Verbindung der Formel VII

      5'-OH(U'')_{m''}(V'')_{n''}O-R₂₂ (VII)

      worin U'' und V'' die Bedeutungen von U und V haben, m'' und n'' unabhängig voneinander 0 oder eine Zahl von 1 bis 198 darstellen, wobei die Summe aus m'' und n'' 2 bis 198 ist, und R₂₂
      (i) einen Rest der Formel IVb bedeutet, worin Z für Sauerstoff oder Schwefel steht; X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten; und R_{b} für C₁-C₁₂-Alkyl, C₆-C₁₂-Aryl, C₇-C₂₀-Aralkyl, C₇-C₂₀-Alkaryl steht, wobei Alkyl, Aryl, Aralkyl und Alkaryl in R_{b} unsubstituiert oder mit Alkoxy, Alkylthio, Halogen, -CN, -NO₂, Phenyl, Nitrophenyl oder Halogenphenyl substituiert sind;
      (ii) eine Hydroxyschutzgruppe bedeutet; oder
      (iii) eine Verknüpfung an ein festes Trägermaterial mit Hilfe einer Brückengruppe bedeutet;
(b) gegebenenfalls Wiederholung von Schritt (a) bis ein Oligonukleotid der gewünschten Länge entstanden ist, wobei vor jeder neuen Ankopplung die Schutzgruppe R₂₀ entfernt, eventuell vorhandene freie Hydroxylgruppen (capping) blockiert und dann das gebildete Phosphit zum Phosphat oxidiert wird,
(c) gegebenenfalls Ablösung des Oligonukleotids oder Isolierung und
(d) Abtrennung der verbliebenen Schutzgruppe R₂₀,
   wobei die Schutzgruppe R₂₀ TTTr ist.

In einer ganz besonders bevorzugten Ausführungsform werden Oligonukleotide der Formel V hergestellt, worin die Summe aus m und n eine Zahl von 2 bis 50, bevorzugt 2 bis 30 ist. Die Nukleoside im Oligonukleotid sind im allgemeinen durch Estergruppen verknüpft. Beispiele sind Phosphorthioate, Phosphordithioate, Phosphoramidate, Alkylphosphonate, Hydrogenphosphonate, Phosphate, Carbonate und Carbamate.

Das Verfahren kann als Festphasenverfahren oder als Verfahren in Lösung durchgeführt werden. Beide Verfahren sind an sich bekannt und werden im allgemeinen bei Temperaturen von -20°C bis 100°C, bevorzugt 10°C bis 60°C durchgeführt. Es ist zweckmässig, die Reaktion in Anwesenheit inerter Lösungsmittel durchzuführen. Beispiele für inerte Lösungsmittel sind Acetonitril, Pyridin, Dioxan, Dimethylformamid, Ethylenglykoldimethylether, Diethylenglykoldimethylether, Chloroform, Trichlorethan, Dichlormethan, N-Methylpyrrolidon, Tetrahydrofuran, Dibutylether und Diethylether. Wenn das Verfahren als Festphasenverfahren durchgeführt wird, erfolgt die Synthese vorteilhafterweise mit einem kommerziell erhältlichen DNA-Synthesizer mit den vom jeweiligen Hersteller beschriebenen und ebenfalls erhältlichen Reagenzien, d.h. Lösungsmittel, Capping-Lösung, Oxidationslösung, Kopplungsreagenz und Detritylierungslösung sowie eine vorbereitete Verbindung der Formel VII.

Unter Hydroxyschutzgruppen sind im Zusammenhang mit R₂₁ die bereits bei den Nukleinbasen genannten Schutzgruppen mit Ausnahme der Amidine zu verstehen.

Feste Trägermaterialien sind bereits weiter vorn angegeben worden. Die fertigen Oligonukleotide können beispielsweise mittels wässriger gesättigter Ammoniaklösung vom Trägermaterial entfernt werden. Dabei werden die Oligonukleotide an ihren Nukleinbasen entschützt. Dieses Verfahren ist beispielsweise auch zur gleichzeitigen Abspaltung der Cycloalkylcarbonsäure-Schutzgruppe geeignet.

Die TTTr-Schutzgruppe wird vorteilhafterweise unter sauren Bedingungen entfernt, beispielsweise mit Dichloressigsäure, Trichloressigsäure, Trifluoressigsäure, Essigsäure, ZnBr₂, Bortrifluoriddietherat.

Die Blockierung eventuell vorhandener freier Hydroxylgruppen erfolgt analog bekannter Verfahren, beispielsweise unter Verwendung von Acetanhydrid, N-Methylimidazol oder 2,6-Lutidin.

Die Oxidation des Phosphits zum Phosphat wird vorteilhafterweise mit einer wässrigen Jodlösung oder Tert.butylhydroperoxid in Acetonitril durchgeführt.

Die Isolierung des fertigen Oligonukleotids kann chromatographisch oder elektrophoretisch erfolgen, von Vorteil ist die Isolierung mittels reversed phase HPLC.

Ein weiterer Gegenstand der vorliegenden Erfindung ist die Verwendung der erfindungsgemässen Nukleoside, Nukleosidanalogen, Nukleotide, Nukleotidanalogen und Oligonukleotide in einem Verfahren zur Herstellung von Oligonukleotiden der Formel V.

Besondere Vorteile hat die Verwendung der TTTr-Gruppe bei der Reinigung von Oligonukleotiden. Es hat sich ausserdem gezeigt, dass sich die erfindungsgemäss geschützten Oligonukleotide durch ihre grössere Stabilität in wässrigen, neutralen Puffersystemen auszeichnen als die mit den gebräuchlichen 4,4'-Dimethoxytritylgruppen geschützten Oligonukleotide, wodurch die Detritylierungsverluste bei der Reinigung, insbesondere der HPLC-Reinigung geringer sind.

Die nachfolgenden Beispiele erläutern die Erfindung näher.

### A: Herstellung der Ausgangsverbindungen

### Beispiel A1: Darstellung von N²-Cyclohexylcarbonyl-2'-desoxyguanosin

28 g Desoxyguanosin werden dreimal mit je 200 ml absolutem Pyridin aufgenommen und jeweils anschliessend im Vakuum bei 50°C zur Trockne eingedampft. Das Nukleosid wird mit 340 ml Diisopropylethylamin aufgenommen und mit 127 ml Trimethylchlorsilan versetzt. Nach zweistündigem Rühren bei Raumtemperatur wird binnen einer Stunde eine Lösung aus 100 ml absolutem Tetrahydrofuran (THF) und 20,4 ml Cyclohexancarbonsäurechlorid hinzugegeben. Die Reaktionsmischung wird nach 16 Stunden auf 0°C abgekühlt und binnen einer Stunde mit 100 ml Methanol versetzt. Anschliessend werden 180 ml einer wässrigen 35 % igen Ammoniumfluoridlösung zugegeben. Eine Stunde nach der Fluoridzugabe werden die flüchtigen Bestandteile im Vakuum bei als 50°C abgedampft. Restliches Pyridin wird durch zweimalige Coevaporation mit je 200 ml Toluol entfernt. Das Rohprodukt wird mit 700 ml Eiswasser 30 Minuten lang verrührt und dann filtriert. Der Filterkuchen wird in 700 ml einer siedenden Lösung aus Ethanol/Wasser 7:3 V/V behandelt. Der unlösliche Anteil wird heiss abfiltriert. Die Titelverbindung fällt bei Abkühlung aus, wird abfiltriert und im Vakuum getrocknet.
¹H-NMR:(DMSO) 8,14: s, H⁸; 6,21: t, 1'; 5,34, 4,97: 2H s (breit) OH; 4,88: m, 3'; 3,8: m, 4'; 2,5: 2H: 2'-, H1 von CC-Gruppe; 2,26: m, 2'; 1,1-1,9: 10H, m, CC-Gruppe.

### Beispiel A2: Darstellung von N⁴-Cyclohexylcarbonyl-2'-desoxycytidin

5,3 g Desoxycytidin werden dreimal in je 100 ml absolutem Pyridin aufgenommen und im Hochvakuum bei 55°C zur Trockne eingeengt. Das so getrocknete Edukt wird in 100 ml absolutem Pyridin aufgenommen und mit 25,4 ml Trimethylchlorsilan versetzt. Nach zweistündigem Rühren wird ine Lösung aus 6,3 g Cyclohexancarbonsäurechlorid in 40 ml absoluten THF zugetropft. Man lässt für 18 Stunden bei Raumtemperatur rühren. Unter Eiskühlung werden dann 20 ml Methanol langsam zugegeben. Die Desilylierung erfolgt durch die Zugabe von 36 ml Wasser einer 35 %igen wässrigen Ammoniumfluoridlösung. Nach 60 Minuten wird die Reaktionsmischung am Vakuum zur Trockne bei 55°C eingeengt. Anhaftendes Pyridin wird durch zweimalige Coevaporation mit je 100 ml Wasser entfernt. Das Rohprodukt wird in 100 ml Wasser aufgenommen und dreimal mit je 100 ml n-Butanol extrahiert. Die Butanolphasen werden vereinigt und nach Trocknung über Natriumsulfat zu einem Öl im Vakuum bei 55°C eingedickt. Das Öl wird mit 500 ml Ether für 5 Stunden verrührt, wobei die gereinigte Titelverbindung kristallin anfällt. Die Kristalle werden abgesaugt und am Hochvakuum getrocknet.
¹H-NMR (CD₃OD): 8,47 d, H⁶; 7,44: d, H⁵; 6,22: q, 1'; 4,4, 4,03: 3', 4'; 3,7-3,9: 2H, m, 5'; 2,35-2,55: 2H, m, 2', C-1, CC-Gruppe; 2,07-2,25: m, 2'; 1,4-1,9, 1,1-1,65: 10H, m, CC-Gruppe.

### Beispiel A3: Darstellung von N⁶-Cyclohexylcarbonyl-2'-desoxyadenosin

5,4 g Desoxycytidin werden dreimal in je 100 ml absolutem Pyridin aufgenommen und am Hochvakuum bei 55°C zur Trockne eingeengt. Das so getrocknete Edukt wird in 100 ml absolutem Pyridin und 68 ml Diisopropylethylamin aufgenommen. Hierzu gibt man 25,4 ml Trimethylchlorsilan. Nach zweistündigem Rühren wird eine Lösung aus 6,3 g Cyclohexancarbonsäurechlorid in 40 ml absolutem THF zugetropft. Man lässt für 18 Stunden bei Raumtemperatur rühren. Unter Eiskühlung werden dann 20 ml Methanol langsam zugegeben. Die Desilylierung erfolgt durch die Zugabe von 36 ml einer 35 %igen wässrigen Ammoniumfluoridlösung. Nach 60 Minuten wird die Reaktionsmischung am Vakuum zur Trockne bei 55°C eingeengt. Das Rohprodukt wird in 100 ml Ethylacetat aufgenommen und sukzessive mit je 100 ml wässrigen Natriumhydrogencarbonates und Sole extrahiert. Die organische Phase wird nach Trocknung über Natriumsulfat zu einem Öl im Vakuum bei 55°C eingedickt. Die Titelverbindung wird durch Fällen mit Diethylether gewonnen.
¹H-NMR (CDCl₃): 8,35, 8,33, 2s, Adenin; 6,25: t, 1'; 4,34: m, 3'; 3,78: m, 4'; 3,5: 2H, m, 5'; 2,55, 2,4, 2,2: 3H, 3m, 2', C-1 CC-Gruppe; 1,85-1,1: 10H: m, CC-Gruppe.

Die Stabilität des N⁶-Cyclohexylcarbonyl-2'-desoxyadenosins gegenüber der Jodoxidationslösung für die DNA-Synthese nach dem Phosphittriesterverfahren: 1 g N⁶-Cyclohexancarbonyl-2'-desoxyadenosin werden für 22 Stunden einer Lösung aus 3 % Jod, 2 % Wasser, 20 % Pyridin und 75 % THF ausgesetzt. Nach dieser Zeit zeigt ein Dünnschichtchromatogramm der Reaktionsmischung keine Umsetzung.

### B: Herstellung der erfindungsgemässen Verbindungen

### Beispiel B1: Darstellung von 5'-(Tris-4,4',4''-tert.butyltrityl)-N²-cyclohexylcarbonyl-2'-desoxyguanosin

27 g N²-Cyclohexylcarbonyl-2'-desoxyguanosin werden dreimal mit je 200 ml absolutem Pyridin aufgenommem und anschliessend im Vakuum bei 50°C zur Trockne eingedampft. Das Nukleosid wird in 600 ml absolutem Pyridin aufgenommen und mit 100 ml Triethylamin sowie anschliessend mit 34,4 g Tris-4,4',4''-tert.butyltritylchlorid versetzt. Es wird 18 Stunden gerührt, wobei ein Niederschlag ausfällt, der abfiltriert wird. Das Filtrat wird zur Trockne im Vakuum bei 50°C eingedampft. Anschliessend wird es in 600 ml Dichlormethan aufgenommen und zweifach mit je 500 ml kalt gesättigter Natriumhydrogencarbonatlösung, dann mit 500 ml Sole extrahiert. Die organische Phase wird über Natriumsulfat getrocknet und nach Abfiltrieren des Salzes zur Trockne bei 100 bis 200 Torr eingeengt. Anschliessend wird das Produkt in 100 ml heissem Methanol suspendiert. Die Suspension wird abfiltriert und das Filtrat auf 0°C abgekühlt, wobei erneut Produkt ausfällt. Nach innigem Verrühren mit 500 ml Petrolether für 2 Stunden wird die Titelverbindung abgesaugt und bei 60°C im Trockenschrank bei 30 bis 50 Torr getrocknet.
¹H-NMR:(CD₃OD) 7,95: H⁸, 7,15: 12H, TTTr; 6,22: t,1'; 4,47: m, 3' ; 3,95: m, 4'; 2,65: m, 2'; 2,2-2,45: m, 2', H1(CC-Gruppe); 1,8-1,1: 10H, m, CC-Gruppe; 1,13: 27H, 2s, TTTr- Gruppe.

### Beispiel B2: Darstellung von 5'-(Tris-4,4',4''-tert.butyltrityl)-N²-cyclohexylcarbonyl-2'-desoxyguanosin-3'-(cyanoethyl)phosphordiisopropylamidit

Zu 27,7 g 5'-(Tris-4,4',4''-tert.butyltrityl)-N²-cyclohexylcarbonyl-2'-desoxyguanosin in 800 ml abolutem Acetonitril werden unter trockenem Argon 20,6 ml Bis-diisopropylamino-cyanoethyl-phosphan und 2,67 g Tetrazol zugesetzt. Es wird 18 Stunden gerührt. 8 ml Triethylamin werden hinzugegeben und das Lösungsmittel im Vakuum bei 50°C abgedampft. Das Rohprodukt wird in 500 ml Ethylacetat aufgenommen und zweimal mit je 500 ml kalt gesättigter Natriumhydrogencarbonatlösung, anschliessend einmal mit 500 ml Sole extrahiert. Die organische Phase wird zur Trockne im Vakuum bei 50°C eingedampft. Man erhält ein Öl, welches zur Darstellung des N-Methylmorpholinium-5'-(tris-4,4',4''-tert.butyltrityl)-N²cyclohexylcarbonyl-2'-desoxyguanosin-3'-(cyanoethyl)phosphorthioats (Beispiel B3) direkt weiterverarbeitet werden kann. Zur Reinigung wird das Rohprodukt der "Flashchromatography" auf Kieselgel unterzogen. Man eluiert das Produkt mit n-Hexan : Ethylacetat : N-Methylmorpholin 30:69:1 V/V/V. Die aus den vereinigten Fraktionen erhaltene Titelverbindung wird im Vakuum bei 50°C eingedampft. Weitere Evaporation von Lösungsmittelresten im Hochvakuum führt zu einem weissen Schaum.
³¹P-NMR (CDCl₃): 148,3, 147,6; ¹H-NMR (CDCl₃): 7,76, 7,72: 2s H⁸(Guanin); 7,38, 7,2 12 H, 2m, TTTr-Gruppe; 6,12, 1'; 4,65: m, 3'; 2,55, 2,6: 2t: CNE 1,22: 27H, s, TTTr-Gruppe; 1,1, 0,98: 12H, i-Propyl (Amidit).

### Beispiel B3: Darstellung von N-Methylmorpholinium-5'-(tris-4,4',4''-tert.butyltrityl)-N²-cyclohexylcarbonyl-2'-desoxyguanosin-3'-(O)-cyanoethylphosphorthioat

Zu 92,2 g 5'-(Tris-tert.butyltrityl)-N²-cyclohexylcarbonyl-2'-desoxyguanosin in 1200 ml absolutem Acetonitril werden unter trockenem Argon 84 ml Bis-diisopropylamino-cyanoethyl-phosphan und 422 ml einer 0,5 M Tetrazollösung zugesetzt. Es wird 18 Stunden gerührt. 16 ml Triethylamin werden hinzugegeben und das Lösungsmittel im Vakuum bei 50°C abgedampft. Das Rohprodukt wird in 1200 ml Ethylacetat aufgenommen und zweimal mit je 1200 ml kalt gesättigter Natriumhydrogencarbonatlösung, anschliessend einmal mit 1200 ml Sole extrahiert. Die organische Phase wird zur Trockne im Vakuum bei 50°C eingedampft. Man erhält ein Öl. Einige Verunreinigungen des Produktes lassen sich durch zehnmaliges Einengen mit je 200 Toluol entfernen, so dass das rohe Phosphoramidit als weisser Schaum anfällt. Das rohe Phosphoramidit wird weiter umgesetzt, wobei zwei Ansätze verwendet und zur Weiterverarbeitung mit 200 g rohen Amidits versetzt werden. Dieses wird in 1,2 l Acetonitril aufgenommen mit 3,7 ml Wasser und 77 g Tetrazol versetzt. Die Reaktionsmischung wird 30 Minuten lang gerührt, anschliessend werden 77 g Schwefel und 1,2 l Pyridin zugegeben. Man lässt die Reaktionslösung 17 Stunden bei Raumtemperatur rühren und engt sie dann im Vakuum bei 50°C zur Trockne ein. Das Rohprodukt wird mit 600 ml einer 1 M wässrigen N-Methylmorpholiniumhydrogencarbonatlösung und 600 ml Ethylacetat versetzt und der ausfallende Schwefel abfiltriert. Man extrahiert die organische Phase erneut zweimal mit je 600 ml einer 1 M wässrigen N-Methylmorpholiniumhydrogencarbonatlösung. Die organische Phase wird über Natriumsulfat getrocknet und zur Trockne im Vakuum bei 50°C eingedampft. Das Rohprodukt wird nun der Flashchromatographie unterzogen, wobei eine Säule aus 4 kg Kieselgel (230-400 Mesh) verwendet wird. Man eluiert das Produkt mit Ethylacetat : Methanol : N-Methylmorpholin 90:9:1, V/V/V. Die vereinigten Produktfraktionen werden zur Trockne im Vakuum bei 50°C eingeengt. Das so erhaltene Öl wird mit 500 ml n-Pentan für 5 Stunden verrührt, wobei die pulvrige Titelverbindung entsteht.
³¹P-NMR(DCCl₃):57,21, 56,80 ppm.

### Beispiel B4: Darstellung von 5'-(Tris-4,4',4''-tert.butyltrityl)-thymidin

3,6 g Thymidin werden je dreimal mit 25 ml absolutem Pyridin versetzt und im Vakuum bei 55°C eingeengt. Dem so getrockneten Produkt gibt man eine Lösung aus 14,8 g Tris-4,4',4''-tert.butyltritylchlorid in 25 ml Dichlormethan bei 0°C binnen einer Stunde hinzu. Nach 18 Stunden Rühren bei Raumtemperatur wird die Reaktionsmischung am Hochvakuum zur Trockne eingeengt. Der so erhaltene weisse Schaum wird in 100 ml Dichlormethan aufgenommen und zweifach mit je 100 ml kalt gesättigter wässriger Natriumhydrogencarbonatlösung, dann mit 100 ml Sole extrahiert. Die organische Phase wird über Natriumsulfat getrocknet, dann zu einem weissen Schaum eingeengt. Jener wird aus einem Gemisch umkristallisiert, das aus Methanol und Wasser 70:30 V/V besteht. Die sich abscheidenden weissen Kristalle werden abgesaugt und am Hochvakuum getrocknet.
¹H-NMR: 8,12:N³-H; 7,65: H-6; 7,25: 12H, m, TTTr-Gruppe; 6,45: t,1'; 4,66: m, 3'; 4,03: 4'; 3,52-3,38: 2H, m, 5'; 2,4: m, 2'; 1,56: 3H, CH₃⁵; 1,3: 27H, s, TTTr-Gruppe.

### Beispiel B5: Darstellung von 5'-(Tris-4,4',4''-tert.butyltrityl)-thymidin-3'-(cyanoethyl)phosphordiisopropylamidit

57 g 5'-(Tris-4,4',4''-tert.butyl-trityl)-thymidin werden dreimal mit je 25 ml absolutem Pyridin versetzt und im Hochvakuum bei 55°C eingeengt. Das so getrocknete Edukt wird in 1 l absolutem Acetonitril aufgenommen. Zu dieser Lösung gibt man 50 ml Bis-diisopropylamino-cyanoethyl-phosphan und 7,9 g (113 mmol) Tetrazol hinzu. Die Reaktionsmischung lässt man 17 Stunden rühren. Anschliessend werden 38 ml Triethylamin hinzugegeben. Die Reaktionsmischung wird unter Vakuum bei 50°C eingeengt. Das so erhaltene Öl wird in 500 ml Ethylacetat aufgenommen und zweifach mit je 500 ml kalt gesättigter wässriger Natriumhydrogencarbonatlösung, dann mit 500 ml Sole extrahiert. Die organische Phase wird über Natriumsulfat getrocknet, dann zu einem Öl eingeengt. Dieses wird in 1 l einer Lösung aus 99 % Methanol und 1 % N-Methylmorpholin gelöst. Unter stetigem Rühren und unter Eiskühlung wird langsam so viel Wasser zugetropft, dass das Produkt in Form weisser Kristalle ausfällt. Die Titelverbindung wird abfiltriert und im Hochvakuum über P₄O₁₀/KOH getrocknet.
¹H-NMR (CDCl₃): 7,75: 2 s, H⁶; 7,33: 12H, s, TTTr-Gruppe; 6,48: m, 1' ; 4,78: m, 3'; 4,16, 4,23: 2m, 4'; 3,95-3,3: 2H, m: CNE-Gruppe, 2H, m: i-Pr (Amidit), 2H, m: 5'; 2,9-2,4: 4H: 2H, m: 2', 2H, 2t, CNE-Gruppe; 1,45-1,15: 42H: t-Bu, TTTr-Gruppe, CH₃, Amidit, CH₃⁵; ³¹P-NMR: 148,4, 148,2.

### Beispiel B6: Darstellung von 5'-(Tris-4,4',4''-tert.butyltrityl)-N²-cyclohexylcarbonyl-2'-desoxyguanosin-3'-(O)-cyanoethyl-(S) 2,4-dichlorbenzylphosphorthioat

1,4 g N-Methylmorpholinium- 5'-(Tris-4,4',4''-tert.butyltrityl)-N²cyclohexancarbonyl-2'-desoxyguanosin-3'-(O)-cyanoethylphosphorthioat werden unter Lichtausschluss mit 0,8 ml 2,6-Lutidin und 1,95 ml 2,4-Dichlorbenzylchlorid in 50 ml Chloroform bei Raumtemperatur 4 Tage gerührt. Das Produktgemisch wird unter Vakuum bei 50°C zu einem Öl konzentriert. Dieses wird in 100 ml Dichlormethan aufgenommen und sukzessive zweimal mit wässriger gesättigter Natriumhydrogencarbonatlösung und einmal mit 100 ml Sole gewaschen. Die über Natriumsulfat getrocknete organische Phase wird im Vakuum zu einem Öl eingeengt. Dieses wird mit Petrolether/Pyridin für fünf Stunden verrührt. Die dabei entstehende feste Titelverbindung wird abgesaugt und über P₄O₁₀/KOH getrocknet.
³¹P-NMR (CDCl₃): 27,0, 27,6 ppm; PD-MS: [M-DCBn⁺]⁻= 936; [M+Na⁺]⁺=1120; [TTTr]⁺=412; [G^{CC}]⁺=263

### Beispiel B7: Darstellung von 5'-(Tris-4,4',4''-tert.butyltrityl)thymidin-3'-(O)-cyanoethyl-(S)-2,4-dichlorbenzylphosphorthioat

2,7 g N-Methylmorpholinium 5'-(Tris-4,4',4''-tert.butyltrityl)-thymidin-3'-(O)-cyanoethylphosphorthioat werden unter Lichtausschluss mit 1,8 ml 2,6-Lutidin und 4,4 ml 2,4-Dichlorbenzylchlorid in 35 ml Chloroform bei Raumtemperatur 4 Tage gerührt. Das Gemisch wird unter Vakuum bei 50°C zu einem Öl konzentriert. Dieses wird in 100 ml Dichlormethan aufgenommen und sukzessive zweimal mit wässriger gesättigter Natriumhydrogencarbonatlösung und einmal mit 100 ml Sole gewaschen. Die über Natriumsulfat getrocknete organische Phase wird im Vakuum zu einem Öl eingeengt. Dieses wird mit Petrolether/Pyridin für fünf Stunden verrührt. Die dabei entstehende feste Titelverbindung wird abgesaugt und über P₄O₁₀/KOH getrocknet.
³¹P-NMR (DCCl₃): 27,42; 27,24; ¹H-NMR (CDCl₃): 8,6: NH; 7,52: H⁶; 7,16: 15H, m, TTTr; 6,45: m, 1'; 5,23: m, 3'; 3,95-4,15: 4H, m, CNE-Gruppe, DCBn-Gruppe; 3,32: 2H: m, 5'; 2,3-2,65: 4H, m, CNE-Gruppe; 2'; 1,2: 30H, 2s, TTTr-Gruppe, CH₃⁵.

8 g 5'-(Tris-4,4',4''-tert.butyltrityl)thymidin-3'-(O)-cyanoethyl-(S)-2,4-dichlorbenzylphosphorthioat werden in 25 ml 3 % Dichloressigsäure in Dichlormethan aufgenommen und auf eine "Flashchromatographiesäule" mit Kieselgel aufgezogen. Man eluiert den Tris-4,4',4''-tert.butyltritylmethylether und den Tris-4,4',4''-tert.butyltritylalkohol mit Dichlormethan : Methanol : DCA, 98 : 1 : 1 (V/V/V). Danach wird das Produkt mit Dichlormethan: Methanol, 9 : 1 (V/V) eluiert. Die Fraktionen werden am Vakuum bei 55°C zu einem Öl konzentriert. Anschliessend wird das Öl in 250 ml Ethylacetat aufgenommen, fünfmal mit je 250 ml 1 M wässrigen Natriumacetat dichloracetatfrei gewaschen. Nach Trocknen über Natriumsulfat und Abdampfen des Lösungsmittels im Vakuum wird Thymidin-3'-(O)-cyanoethyl-(S)-2,4-dichlorbenzylphosphorthioat erhalten.
³¹P-NMR (CDCl₃) 28,15, 27,79; ¹H-NMR (CDCl₃): 8,48: NH; 7,45; 7, 17: 4H, DCBn-Gruppe, H⁶; 6,05: m, 1'; 5,13: 3', m; 4,0-4,3: 5H, DCBn-Gruppe, 4', CNE-Gruppe; 3,78: m, 5'; 2,7: m,m, CNE-Gruppe; 2,35: 2'; 1,87: s, CH₃⁵.

### Beispiel B8: Darstellung von Triethylammonium 5'-(Tris-4,4',4''-tert.butyltrityl)thymidin-3'-(S)-dichlorbenzylphosphorthioat

16 g 5'-(Tris-4,4',4''-tert.butyltrityl)thymidin-3'-(O)-cyanoethyl-(S)2,4-dichlorbenzylphosphorthioat werden mit 500 ml einer Lösung aus Acetonitril/Triethylamin 9:1 V/V versetzt. Nach zwei Stunden wird die Lösung im Vakuum eingeengt und für zwei Stunden in 500 ml Ether verrührt. Nach Absaugen und Trocknung am Hochvakuum erhält man die Titelverbindung.
³¹P-NMR (CDCl₃): 16,69; ¹H-NMR (CDCl₃): 7,58: s, H⁶; 7,15: 15H, m, TTTr; 6,34: m, 1'; 5,05: m, 3'; 3,85-4,1: 3H, m, 4', DCBn; 3,15: 5'; 2,92: 6H, q, Triethylamin; 2,22, 2,3: 2'; 0,9-1,35: m, 39H, TTTr, Triethylamin, CH₃⁵.

### Beispiel B9: Darstellung von 5'-(Tris-4,4',4''-tert.butyltrityl)-N⁶-pyrrolidinylmethyliden-2'-desoxyadenosin

233 mg N⁶-pyrrolidinylmethylidendesoxyadenosin werden dreimal mit je 10 ml absolutem Pyridin aufgenommen und dann so im Vakuum zur Trockne eingedampft, dass die Temperatur 50°C nicht übersteigt. Anschliessend wird die Substanz mit 10 ml absolutem Pyridin aufgenommen. Zu dieser Lösung gibt man bei 0°C unter Rühren binnen einer Stunde eine Lösung aus 343,8 mg TTTrCl und 10 ml Dichlormethan. Nach 60 Stunden wird das Reaktionsgemisch auf 2 ml eingeengt und diese anschliessend in 100 ml Wasser einfliessen lassen. Der Niederschlag wird in 100 ml Dichlormethan aufgenommen. Die organische Phase wird zweimal mit je 100 ml wässriger gesättigter Natriumhydrogencarbonatlösung anschliessend mit 100 ml Sole extrahiert. Die gewaschene organische Phase wird über Natriumsulfat getrocknet. Nach Entfernen des Salzes wird das Produkt zur Trockne eingedampft. Der erhaltene Feststoff wird mit 100 ml Petrolether (hochsiedend) für eine Stunde verrieben. Man filtriert das Produkt ab, kühlt die Mutterlauge auf 0°C ab und filtriert erneut. Der Filterkuchen wird anschliessend mit 0°C kaltem Petrolether (hochsiedend) gewaschen. Nach Trocknung im Hochvakuum wird die Titelverbindung erhalten.
¹H-NMR (CDCl₃): 9,28: s, Amidin; 8,62: s, 8,15: s, Adenin; 7,4: 12H, m, TTTr; 6,62:m, 1'; 4,77: m, 3'; 4,22: m 4'; 3,3, 3,82: 2m, 4H Pyrrolidinyl; 3,54: d, 2H, 5'; 2,88, 2,55: 2m, 2'; 2H, 2,06: m, 4H, Pyrrolidinyl; 1,4: s, 27H, TTTr.

### Beispiel B10: Darstellung von 5'-(Tris-4,4',4''-tert.butyltrityl)-N²-dimethylamino-methyliden-2'-desoxy-guanosin

967 mg N²-Dimethylaminomethylidendesoxyguanosin werden dreimal mit je 10 ml absolutem Pyridin aufgenommen und dann so im Vakuum zur Trockne eingedampft, dass die Temperatur 50°C nicht übersteigt. Anschliessend wird die Substanz mit 10 ml absolutem Pyridin aufgenommen. Zu dieser Lösung gibt man bei 0°C unter Rühren binnen einer Stunde eine Lösung aus 2950 mg TTTrCl und 10 ml Dichlormethan. Nach 60 Stunden wird das Reaktionsgemisch auf 2 bis 3 ml eingeengt und diese anschliessend in 100 ml Wasser einfliessen lassen. Der Niederschlag wird in 100 ml Dichlormethan aufgenommen. Die organische Phase wird zweimal mit je 100 ml wässriger gesättigter Natriumhydrogencarbonallösung, anschliessend mit 100 ml Sole extrahiert. Die gewaschene organische Phase wird über Natriumsulfat getrocknet. Nach Entfernen des Salzes wird das Produkt zur Trockne eingedampft. Der erhaltene Feststoff wird mit 100 ml Petrolether (hochsiedend) für eine Stunde verrieben. Man filtriert das Produkt ab, kühlt die Mutterlauge auf 0°C ab und filtriert erneut. Der Filterkuchen wird anschliessend mit 0°C kaltem Petrolether (hochsiedend) gewaschen. Nach Trocknung im Hochvakuum wird die Titelverbindung erhalten.
¹H-NMR (DMSOD₆): 11,2, 8,46, 7,75: 2s, Amidine, Guanin; 7,08: 12H, m, TTTr; 6,12:m, 1'; 5,22: s, OH; 4,37: m, 3'; 3,75: m 4'; 2,96: m, 5H, 5', Amidine; 2,89: s, 3H, Amidine; 2,5, 2,15: 2m, 2'; 1,08: s, 27H, TTTr.

### Beispiel B11: Darstellung von N-(1-Methyl-2-pyrrolidin-methyliden)-5'-(tris-4,4',4''-tert.butyltrityl)adenosin

50 g trockenes Adenosin, 20 g Molekularsieb (3 Å), 800 ml wasserfreies Methanol und 80 ml wasserfreies Pyridin werden bei Raumtemperatur gerührt und 29,04 g destilliertes N-Methylpyrrolidondimethylacetal hinzugefügt. Das Molekularsieb wird abfiltriert. Das Filtrat wird im Vakuum konzentriert. Der Rückstand wird mit je 100 ml Acetonitril dreimal konzentriert und getrocknet. Dieses Material wird in 450 ml wasserfreiem Pyridin gelöst und mit 50 g Molekularsieb (5 Å) versetzt. Die Reaktionsmischung wird bei 60° C gerührt und mit 100,3 g festem Tris-4,4',4''-tert.butyltrityl)chlormethan versetzt. Nach 15 Stunden Rühren wird die Reaktionstemperatur für 5 Stunden auf 70° C erhöht. Das Molekularsieb wird abfiltriert, das Filtrat wird zur Trockne eingeengt. Der Rückstand wird über eine Silikagel-Säule (Essigsäureethylester/Methanol/N-Methylmorpholin 20:2:0,1; Durchmesser 8 cm) gegeben. Die die Titelverbindung enthaltenden Fraktionen werden gesammelt, konzentriert und getrocknet.
¹H-NMR (250 MHz, CDCl₃): 1,27 (s, ca. 27 H, 3 (CH₃)₃C-ar); 2,04 (m, 3H); 2,37 (m, 2H); 2,90-3,15 (m, 2H); 3,18 (s, NCH₃); 3,2-3,3 (m, 2H); 3,39 (t, ca. 1H); 3,51 (t-like m, J = 8, 3 H); 3,69 (s, 2H); 4,37 (d-like m, J = 5, 1 H); 4,45 (br s, 1 H); 4,82 (t, J = 5, 1 H); 5,99 (d, J = 7, H-C(1')); 6,72 (br s, 1 H); 7,20 (s, 12 H); 8,23 (s) und 8,54 (s)(H-C(2,8)).

### Beispiel B12: Darstellung von 5'-(Tris-4,4',4''-tert.butyltrityl)-6-methyluracil

40 mMol 6-Methyluracil werden in 75 ml wasserfreiem Pyridin gelöst. Die Lösung wird auf 60° C erwärmt und mit 44 mMol festem Tris-4,4',4''-tert.butyltrityl)-chlormethan versetzt. Nach 6 Stunden Rühren bei 60° C wird das Lösungsmittel im Vakuum entfernt und der Rückstand wird durch einmal Aufnehmen in Toluol und zweimal in Acetonitril aufkonzentriert. Anschliessend wird in 600 ml Methylchlorid gelöst und mit 200 ml gesättigter NaHCO₂-Lösung gewaschen. Die organische Phase wird über Na₂SO₄ getrocknet und die Lösungsmittel im Vakuum entfernt. Der Rückstand wird in 220 ml heissem Ethanol/Wasser-Gemisch (3:2) gelöst und bei 0° C kristallisiert. Schmelzpunkt: 169-170° C.
¹H-NMR (250 MHz, CDCl₃): 1,29 (s, ca. 27 H, 3 (CH₃)₃C-ar); 1,38 (s, 3H, CH₃C(5)); 3,14 (br. d, 1 H); 3,47 (ABM-System, 2H, H₂C(5')); 4,22 (d-like m, 1H) und 4,47 (m, 2H)(HC(2',3',4'); 5,13 (br. s, 1H); 5,96 (d, J=4, 1H, HC(1')); 7,15-7,35 (m, ca. 12H,H-(ar)); 7,81 (s, HC(6)); 9,75 (br. s, HN(3)).

### Beispiel B13: Darstellung von 5'-(Tris-4,4',4''-tert.butyltrityl)uridin

200 mMol Ribouridin werden in 375 ml wasserfreiem Pyridin gelöst. Die Lösung wird auf 60° C erwärmt und mit 220 mMol festem Tris-4,4',4''-tert.butyltrityl)-chlormethan versetzt. Nach 6 Stunden Rühren bei 60° C wird das Lösungsmittel im Vakuum entfernt und der Rückstand wird durch einmal Aufnehmen in Toluol und zweimal in Acetonitril aufkonzentriert. Anschliessend wird in 600 ml Methylchlorid gelöst und mit 200 ml gesättigter Bicarbonat-Lösung gewaschen. Die organische Phase wird über Na₂SO₄ getrocknet und die Lösungsmittel im Vakuum entfernt. Der Rückstand wird in 220 ml heissem Ethanol/Wasser-Gemisch (3:2) gelöst und bei 0° C kristallisiert.
¹H-NMR (250 MHz, CDCl₃): 1,29 (s, ca. 27 H, 3 (CH₃)₃C-ar); 3,14 (br. d, 1 H); 3,54 (ABM-System, 2H, H₂C(5')); 4,18 (d-like m, 1H), 4,33 (m, 1H) und 4,45 (q-like m, 1H)(HC(2',3',4'); 5,23 (d, J = 8, 1H, HC(5)); 5,35 (br. s, 1H); 5,91 (d, J=2, 1H, HC(1')); 7,15-7,35 (m, ca. 12H,H-(ar)); 8,10 (d, J=8, 1H, HC(6)); 10,06 (br. s, HN(3)).

### C: Anwendungsbeispiele

### Beispiel C1: Darstellung von 5'-(5'-(Tris-4,4',4''-tert.butyltrityl)thymidin-3'-(S)-2,4-dichlorbenzylthioyl)thymidin-3'-(O)-cyanoethyl-(S)-2,4-dichlorbenzyl phosphorthioat

3,2 g Thymidin-3'-(S)-dichlorbenzyl(O)-cyanoethylphosphorthioat und 5,85 g 5'-(Tris-4,4',4''-tert.butyltrityl)thymidin-3'-(O)-cyanoethyl-(S)2,4-dichlorbenzylphosphorthioat löst man in einer Lösung aus 4,25 ml absolutem N-Methylimidazol und 8,1 g Triisopropylbenzolsulfonylchlorid in 125 ml absolutem Pyridin. Nach 2,5 Stunden Rühren bei Raumtemperatur werden 10 ml Wasser zugegeben. Die Reaktionsmischung wird bei 55°C zu einem Öl aufkonzentriert. Man nimmt das Rohprodukt in 500 ml Ethylacetat auf und extrahiert sukzessive zweimal mit je 500 ml wässrigem N-Methylmorpholiniumhydrogencarbonat und einmal mit 500 ml Sole. Die organische Phase wird über Natriumsulfat getrocknet und nach Entfernen des Salzes am Vakuum bei 55°C zur Trockne eingedampft. Nun wird das Produkt in Ether gelöst und in Petrolether gefällt. Nach Filtration und erneutem Verrühren in n-Pentan wird die Titelverbindung als Feststoff erhalten.
³¹P-NMR (CDCl₃): 28,1, 27,8, 27,7, 27,3 (4-Diastereomere); ¹H-NMR (CDCl₃): 8,5-9 NH; 7,58: 1H, H⁶;7,26-7,4:16H, m, DCBn-Gruppe, TTTr-Gruppe, H⁶; 6,44, 6,15: 2H, 2m, 1'; 5,05-5,2, 5,26-5,42: 2H, 2m, 3'; 4,05-4,45: 8H, m, DCBn-Gruppe, CNE-Gruppe, 5', 4',3,3-3,5: 2H, m, 5'; 2,65-2,75, 2,38-2, 6: 6H, CNE-Gruppe, 2'; 1,84: 3H, s, CH₃⁵; 1,3, s, 30 H, TTTr-Gruppe, CH₃⁵.

### Beispiel C2: Verwendung des 5'-(Tris-4,4',4''-tert.butyltrityl)-thymidin-3'-(cyanoethyl)phosphordiisopropylamidits in der automatisierten DNA-Synthese nach dem Phosphittriester-Verfahren

Die Synthese erfolgt mit einem kommerziell erhältlichen DNA-Synthesizer (Applied Biosystems Inc. (ABI)) mit den vom Hersteller beschriebenen Reagenzien, d.h. Acetonitril, Capping-Lösung, Oxidationslösung, Kopplungsreagenz (Tetrazol) und Detritylierungslösung, CPG-DMTrT-Träger. Die vom Hersteller beschriebene Synthesevorschrift wird übernommen bis auf die Detritylierungszeit, welche verdoppelt wird.

Es werden folgende Synthesen durchgeführt: T₁₀, T₂₀ und T₃₀ werden mit dem TTTr-Amidit durchgeführt. Als Vergleich wird die Sequenz T₁₀ mit bekannten DMTr-Amiditen unter Verwendung derselben Synthesereagenzien durchgeführt. Bei allen Oligonukleotidsynthesen wird nach dem letzten Kopplungsschritt die 5'-terminale Schutzgruppe, d.h. die TTTr-Gruppe oder, bei der Kontrollsequenz, die DMTr-Gruppe an dem Polymer belassen. Nach Abschluss der Synthese erfolgt die Ablösung der Oligonukleotide mittels wässriger gesättigter Ammoniaklösung bei 55°C binnen 15 Stunden. Nach Abfiltrieren des CPG-Trägers werden die Produktlösungen lyophilisiert. Nach Aufnehmen in HPLC-Puffer und Feinfiltration werden die Produktgemische über eine kommerzielle C-18-reverse phase Säule aufgetrennt.

Die Analyse der HPLC-Chromatogramme zeigt, dass die TTTr-Amidite sich genauso für die Oligonukleotidsynthese nach dem Phosphittriesterverfahren eignen wie die bekannten DMTr-Amidite. Die TTTr-terminal geschützten Oligonukleotide haben unter gleichen Bedingungen eine höhere Retention auf einer C-18 reversed phase HPLC-Säule. Somit ist im Falle der TTTr-Gruppe nur eine geringe Trennleistung zur Abtrennung der Fehlsequenzen erforderlich.

## Patentansprüche

1. Ein Nukleosid, Nukleosidanaloges, Nukleotid, Nukleotidanaloges oder Oligonukleotid aus mindestens zwei solcher gleichen oder verschiedenen Nukleotide und/oder Nukleotidanalogen, deren Grundgerüst einen gegebenenfalls substituierten Rest einer Nukleinbase B und jeweils eine primäre, geschützte Hydroxylgruppe enthält, dadurch gekennzeichnet, dass die Schutzgruppe Tris-4,4',4''-tert.butyltrityl ist.

2. Ein Nukleosid, Nukleosidanaloges, Nukleotid, Nukleotidanaloges oder Oligonukleotid gemäss Anspruch 1, dadurch gekennzeichnet, dass es zusätzlich mit einer unsubstituierten oder mit C₁-C₆-Alkyl oder C₁-C₆-Alkoxy substituierten Cycloalkylcarbonylgruppe mit 3 bis 12 Ringkohlenstoffatomen oder mit einer Amidinschutzgruppe geschützt ist.

3. Ein Nukleosid, Nukleosidanaloges, Nukleotid, Nukleotidanaloges oder Oligonukleotid gemäss Anspruch 2, dadurch gekennzeichnet, dass die Cycloalkylcarbonylgruppe 4 bis 8 Ringkohlenstoffatome enthält.

4. Ein Nukleosid, Nukleosidanaloges, Nukleotid, Nukleotidanaloges oder Oligonukleotid gemäss Anspruch 3, dadurch gekennzeichnet, dass die Cycloalkylcarbonylgruppe 5 oder 6 Ringkohlenstoffatome enthält.

5. Ein Nukleosid, Nukleosidanaloges, Nukleotid, Nukleotidanaloges oder Oligonukleotid gemäss Anspruch 4, dadurch gekennzeichnet, dass die Cycloalkylcarbonylgruppe Cyclohexancarbonyl ist.

6. Ein Nukleosid, Nukleosidanaloges, Nukleotid, Nukleotidanaloges oder Oligonukleotid gemäss Anspruch 1, worin es sich bei der Nukleinbase B um einen Rest der Formel I, Ia, Ib, Ic, Id oder Ie handelt, 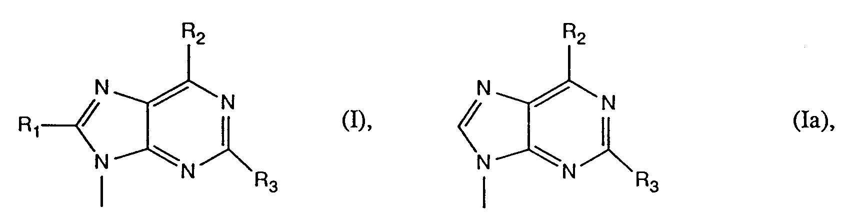 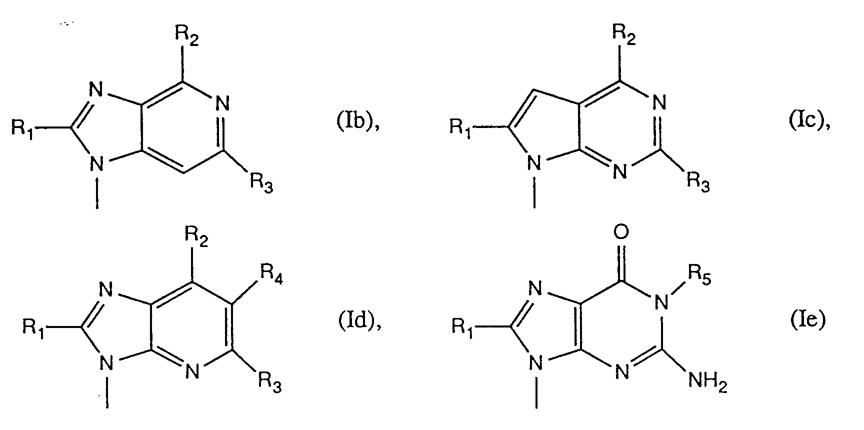 worin R₁ für H, Cl, Br, NH₂ oder OH steht, und R₂, R₃ und R₄ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und R₅ H oder C₁-C₄-Alkyl darstellt.

7. Ein Nukleosid, Nukleosidanaloges, Nukleotid, Nukleotidanaloges oder Oligonukleotid gemäss Anspruch 6, worin das Primäramino 1 bis 12 C-Atome und das Sekundäramino 2 bis 12 C-Atome enthält.

8. Ein Nukleosid, Nukleosidanaloges, Nukleotid, Nukleotidanaloges oder Oligonukleotid gemäss Anspruch 6, worin es sich bei dem Primäramino und Sekundäramino um Reste der Formel R₆R₇N handelt, worin R₆ für H steht oder unabhängig die Bedeutung von R₇ hat, und R₇ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome und die Alkylgruppe 1 bis 6 C-Atome enthält; C₂-C₂₀-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R₆ und R₇ zusammen Tetra- oder Pentamethylen, 3-Oxa-1,5-pentylen, -CH₂-NR₈-CH₂CH₂- oder -CH₂CH₂-NR₈-CH₂CH₂- darstellen, worin R₈ für H oder C₁-C₄-Alkyl steht, wobei die Aminogruppe im Aminoalkyl unsubstituiert oder mit ein oder zwei C₁-C₄-Alkyl oder -Hydroxyalkylgruppen substituiert ist, und die Hydroxylgruppe im Hydroxyalkyl gegebenenfalls mit C₁-C₄-Alkyl verethert ist.

9. Ein Nukleosid, Nukleosidanaloges, Nukleotid, Nukleotidanaloges oder Oligonukleotid gemäss Anspruch 7, worin es sich bei dem Primäramino und Sekundäramino um Methyl-, Ethyl-, Dimethyl-, Diethyl-, Allyl-, Mono- oder Di-(hydroxy-eth-2-yl)-, Phenyl-, Benzyl-, Acetyl-, Isobutyryl und Benzoylamino handelt.

10. Ein Nukleosid, Nukleosidanaloges, Nukleotid, Nukleotidanaloges oder Oligonukleotid gemäss Anspruch 6, worin R₁ in den Formeln I, Ib, Ic, Id und Ie für Wasserstoff steht.

11. Ein Nukleosid, Nukleosidanaloges, Nukleotid, Nukleotidanaloges oder Oligonukleotid gemäss Anspruch 6, worin R₄ in Formel Id für Wasserstoff steht.

12. Ein Nukleosid, Nukleosidanaloges, Nukleotid, Nukleotidanaloges oder Oligonukleotid gemäss Anspruch 6, worin R₂ und R₃ in den Formeln I, Ia, Ib, Ic, Id und Ie unabhängig voneinander H, F, Cl, Br, OH, SH, NH₂, NHOH, NHNH₂, Methylamino, Dimethylamino, Benzoylamino, Methoxy, Ethoxy und Methylthio darstellen.

13. Ein Nukleosid, Nukleosidanaloges, Nukleotid, Nukleotidanaloges oder Oligonukleotid gemäss Anspruch 6, worin B ein Purinrest oder ein Rest eines Purinanalogen aus der Reihe Adenin, N-Methyladenin, N-Benzoyladenin, 2-Methylthioadenin, 2-Aminoadenin, 2-Hydroxypurin, 2-Amino-6-chlorpurin, 2-Amino-6-methylthiopurin, Guanin und N-Isobutyrylguanin ist.

14. Ein Nukleosid, Nukleosidanaloges, Nukleotid, Nukleotidanaloges oder Oligonukleotid gemäss Anspruch 1, wobei es sich bei der Nukleinbase B um einen Rest der Formel II, IIa oder IIb handelt, 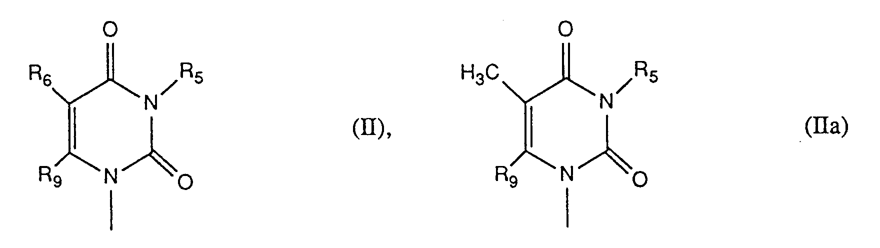 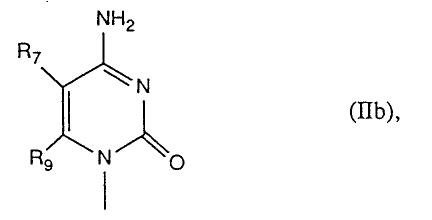 worin R₅ H oder C₁-C₄-Alkyl bedeutet, und R₆, R₇ und R₉ unabhängig voneinander H, OH, SH, NH₂, NHNH₂, NHOH, NHOAlkyl mit 1 bis 12 C-Atomen, F, Cl, Br, Alkyl oder Hydroxyalkyl oder Aminoalkyl oder Alkoxy oder Alkylthio mit 1 bis 12 C-Atomen, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, und die Wasserstoffatome der NH₂-Gruppe in Formel IIb mit C₁-C₆-Alkyl oder Benzoyl substituiert sein können, sowie deren Dihydroderivate.

15. Ein Nukleosid, Nukleosidanaloges, Nukleotid, Nukleotidanaloges oder Oligonukleotid gemäss Anspruch 14, worin R₆ H, C₁-C₆-Alkyl oder -Hydroxyalkyl, F, Cl, Br, NH₂, Benzoylamino, Mono- oder Di-C₁-C₆-alkylamino darstellt.

16. Ein Nukleosid, Nukleosidanaloges, Nukleotid, Nukleotidanaloges oder Oligonukleotid gemäss Anspruch 14, worin R₇ H, C₁-C₆-Alkyl oder -Alkoxy oder -Hydroxyalkyl, F, Cl, Br, NH₂, Benzoylamino, Mono- oder Di-C₁-C₆-alkylamino darstellt.

17. Ein Nukleosid, Nukleosidanaloges, Nukleotid, Nukleotidanaloges oder Oligonukleotid gemäss Anspruch 16, worin R₇ H, F, Cl, Br, NH₂, NHCH₃, N(CH₃)₂ oder C₁-C₄-Alkyl bedeutet.

18. Ein Nukleosid, Nukleosidanaloges, Nukleotid, Nukleotidanaloges oder Oligonukleotid gemäss Anspruch 16, worin R₇ H, C₁-C₄-Alkyl, NH₂, NHCH₃ oder (CH₃)₂N bedeutet.

19. Ein Nukleosid, Nukleosidanaloges, Nukleotid, Nukleotidanaloges oder Oligonukleotid gemäss Anspruch 14, worin B ein Pyrimidinrest oder ein Rest eines Pyrimidinanalogen aus der Reihe Uracil, Thymin, Cytosin, 5-Fluoruracil, 5-Chloruracil, 5-Bromuracil, Dihydrouracil oder 5-Methylcytosin ist.

20. Ein Nukleosid, Nukleosidanaloges, Nukleotid, Nukleotidanaloges oder Oligonukleotid gemäss Anspruch 1, dadurch gekennzeichnet, dass es eine der nachfolgenden Formeln IIIa, IIIb, IIIc oder IIId 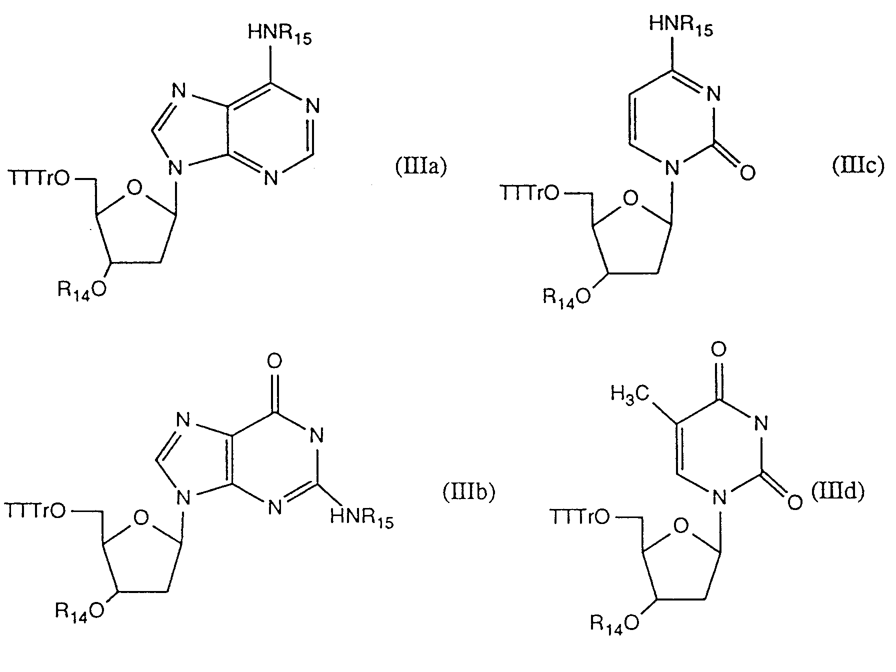 aufweist,
worin R₁₄ Wasserstoff oder einen Nukleotid-Brückengruppe bildenden Rest und R₁₅ Wasserstoff oder Cyclohexylcarbonyl bedeuten.

21. Ein Nukleosid, Nukleosidanaloges, Nukleotid, Nukleotidanaloges oder Oligonukleotid gemäss Anspruch 20, dadurch gekennzeichnet, dass R₁₄ einen phosphorhaltigen, eine Nukleotid-Brückengruppe bildenden Rest der Formel IVa, IVb oder IVc 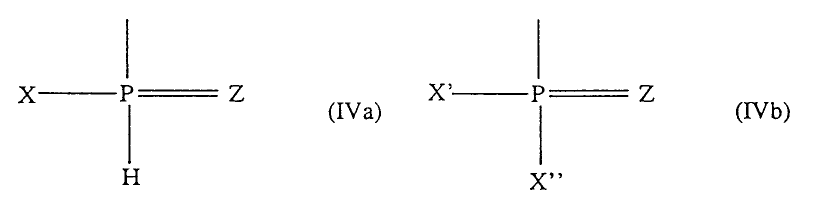 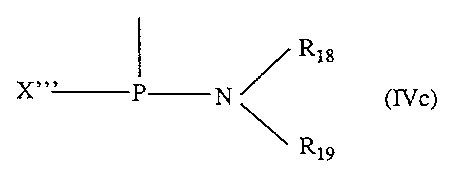 bedeutet, worin
Z Sauerstoff oder Schwefel bedeutet;
X, X' und X'' unabhängig voneinander Sauerstoff oder Schwefel mit negativer Ladung bedeuten, mit Gegenion Li, Na, K, Cs, Tertiär- oder Quartenärammonium; oder
X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten;
X''' C₁-C₁₂-Alkyl, C₆-C₁₂-Aryl, C₇-C₂₀-Aralkyl, C₇-C₂₀-Alkaryl, -OR_{b} oder -SR_{b}, bedeutet; und
R_{b} C₁-C₁₂-Alkyl, C₆-C₁₂-Aryl, C₇-C₂₀-Aralkyl, C₇-C₂₀-Alkaryl bedeutet;
R₁₈ und R₁₉ unabhängig voneinander Wasserstoff, C₁-C₁₂-Alkyl, C₂-C₁₂-Alkenyl, C₆-C₁₂-Aryl, C₇-C₂₀-Aralkyl oder C₇-C₂₀-Alkaryl bedeuten;
wobei Alkyl, Aryl, Aralkyl und Alkaryl in R₁₈, R₁₉ und R_{b} unsubstituiert oder mit Alkoxy, Alkylthio, Halogen, -CN, -NO₂, Phenyl, Nitrophenyl oder Halogenphenyl substituiert sind.

22. Verfahren zur Herstellung von Nukleosiden und Nukleosidanalogen gemäss Anspruch 1, dadurch gekennzeichnet, dass ein Nukleosid oder Nukleosidanaloges mit Tris-4,4',4''-tert.-butyltritylchlorid in Anwesenheit eines sterisch gehinderten tertiären Amins in einem geeigneten aprotischen, polaren, basischen Lösungsmittels umgesetzt wird.

23. Ein Verfahren zur Herstellung von Oligonukleotiden der Formel V
5'-OH(U)ₘ(V)ₙOH-3' (V)
worin U und V gleiche oder verschiedene, natürliche oder synthetische Nukleosidreste bedeuten und m und n unabhängig voneinander 0 oder eine Zahl von 1 bis 200 darstellen, wobei die Summe aus m und n 2 bis 200 ist, durch
(a) Umsetzung einer Verbindung der Formel VI
R₂₀-O-(U')_{m'}(V')_{n'}OR₂₁-3' (VI)
worin R₂₀ eine Schutzgruppe ist und U' und V' die Bedeutungen von U und V haben, m' und n' unabhängig voneinander 0 oder eine Zahl von 1 bis 199 darstellen, wobei die Summe aus m' und n' 2 bis 199 ist, und R₂₁ einen phosphorhaltigen, eine Nukleotid-Brückengruppe bildenden Rest der Formel IVa, IVb oder IVc 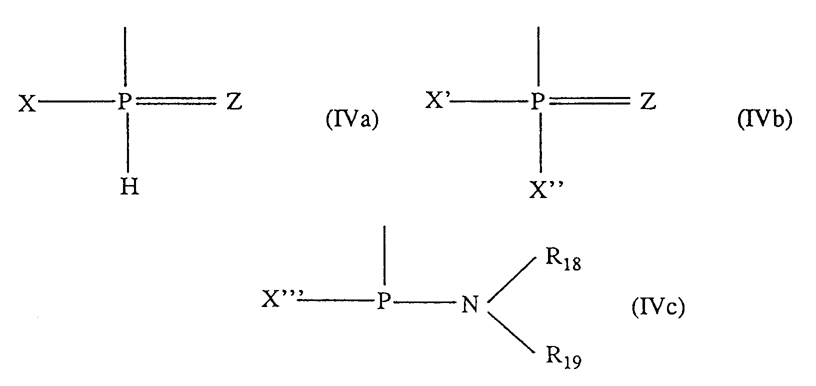 bedeutet, worin
Z Sauerstoff oder Schwefel bedeutet;
X, X' und X'' unabhängig voneinander Sauerstoff oder Schwefel mit negativer Ladung bedeuten, mit Gegenion Li, Na, K, Cs, Tertiär- oder Quartenärammonium; oder
X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten;
X''' C₁-C₁₂-Alkyl, C₆-C₁₂-Aryl, C₇-C₂₀-Aralkyl, C₇-C₂₀-Alkaryl, -OR_{b} oder -SR_{b}, bedeutet; und
R_{b} C₁-C₁₂-Alkyl, C₆-C₁₂-Aryl, C₇-C₂₀-Aralkyl, C₇-C₂₀-Alkaryl bedeutet;
R₁₈ und R₁₉ unabhängig voneinander Wasserstoff, C₁-C₁₂-Alkyl, C₂-C₁₂-Alkenyl, C₆-C₁₂-Aryl, C₇-C₂₀-Aralkyl oder C₇-C₂₀-Alkaryl bedeuten;
wobei Alkyl, Aryl, Aralkyl und Alkaryl in R₁₈, R₁₉ und R_{b} unsubstituiert oder mit Alkoxy, Alkylthio, Halogen, -CN, -NO₂, Phenyl, Nitrophenyl oder Halogenphenyl substituiert sind,
mit einer Verbindung der Formel VII
5'-OH(U'')_{m''}(V'')_{n''}O-R₂₂ (VII)
worin U'' und V'' die Bedeutungen von U und V haben, m'' und n'' unabhängig voneinander 0 oder eine Zahl von 1 bis 198 darstellen, wobei die Summe aus m'' und n'' 2 bis 198 ist, und R₂₂
(i) einen Rest der Formel IVb bedeutet, worin Z für Sauerstoff oder Schwefel steht; X' und X'' unabhängig voneinander C₁-C₁₂-Alkyl, C₆-C₁₂-Aryl, C₇-C₂₀-Aralkyl, C₇-C₂₀-Alkaryl, -OR_{b} oder -SR_{b} bedeuten; und R_{b} für C₁-C₁₂-Alkyl, C₆-C₁₂-Aryl, C₇-C₂₀-Aralkyl, C₇-C₂₀-Alkaryl steht, wobei Alkyl, Aryl, Aralkyl und Alkaryl in R_{b} unsubstituiert oder mit Alkoxy, Alkylthio, Halogen, -CN, -NO₂, Phenyl, Nitrophenyl oder Halogenphenyl substituiert sind;
(ii) eine Hydroxyschutzgruppe bedeutet; oder
(iii) eine Verknüpfung an ein festes Trägermaterial mit Hilfe eines "linkers" bedeutet;
(b) gegebenenfalls Wiederholung von Schritt (a) bis ein Oligonukleotid der gewünschten Länge entstanden ist, wobei vor jeder neuen Ankopplung die Schutzgruppe R₂₀ entfernt, eventuell vorhandene freie Hydroxylgruppen (capping) blockiert und dann das gebildete Phosphit zum Phosphat oxidiert wird,
(c) gegebenenfalls Ablösung des Oligonukleotids oder Isolierung und
(d) Abtrennung der verbliebenen Schutzgruppe R₂₀, dadurch gekennzeichnet, dass die Schutzgruppe R₂₀ TTTr ist.

24. Ein Verfahren gemäss Anspruch 23, dadurch gekennzeichnet, dass die Summe aus m und n eine Zahl von 2 bis 50 ist.

25. Ein Verfahren gemäss Anspruch 24, dadurch gekennzeichnet, dass die Summe aus m und n eine Zahl von 2 bis 30 ist.

26. Verwendung eines Nukleotids, Nukleotidanalogen oder Oligonukleotids gemäss Anspruch 1 zur Herstellung von Oligonukleotiden der Formel V
5'-OH(U)ₘ(V)ₙOH-3' (V)
worin U und V gleiche oder verschiedene, natürliche oder synthetische Nukleosidreste bedeuten und m und n unabhängig voneinander 0 oder eine Zahl von 1 bis 200 darstellen, wobei die Summe aus m und n 2 bis 200 ist.

27. Eine Verbindung gemäss Anspruch 2, dadurch gekennzeichnet, dass die Amidinschutzgruppe Dimethylaminomethyliden ist.

28. Nukleosid, Nukleosidanaloges, Nukleotid, Nukelotidanaloges oder Oligonukleotid gemäss Anspruch 1, dadurch gekennzeichnet, dass sich die primäre, geschützte Hydroxylgruppe an der 5'-Position des Grundgerüstes befindet.

29. Verwendung des Restes Tris-4,4',4''-tert.butyltrityl als Schutzgruppe für eine primäre Hydroxylgruppe des Grundgerüstes von Nukleosiden, Nukleosidanalogen, Nukleotiden, Nukleotidanalogen oder Oligonukleotiden aus mindestens zwei solcher gleichen oder verschiedenen Nukleotide und/oder Nukleotidanalogen, deren Grundgerüst einen gegebenenfalls substituierten Rest einer Nukleinbase B und jeweils eine primäre Hydroxylgruppe enthält.

30. Verwendung gemäss Anspruch 29, dadurch gekennzeichnet, dass sich die primäre Hydroxylgruppe an der 5'-Position des Grundgerüstes befindet.

## Claims

1. A nucleoside, nucleoside analog, nucleotide, nucleotide analog or oligonucleotide comprising at least two such identical or different nucleotides and/or nucleotide analogs, whose parent structure comprises an unsubstituted or substituted residue of a nucleobase B and one primary, protected hydroxyl group, wherein the protective group is tris-4,4',4''-tert-butyltrityl.

2. A nucleoside, nucleoside analog, nucleotide, nucleotide analog or oligonucleotide according to claim 1, which is additionally protected by an amidine protective group or by a cycloalkylcarbonyl group which contains 3 to 12 ring carbon atoms and which is unsubstituted or substituted by C₁-C₆alkyl or C₁-C₆alkoxy.

3. A nucleoside, nucleoside analog, nucleotide, nucleotide analog or oligonucleotide according to claim 2, wherein the cycloalkylcarbonyl group contains 4 to 8 ring carbon atoms.

4. A nucleoside, nucleoside analog, nucleotide, nucleotide analog or oligonucleotide according to claim 3, wherein the cycloalkylcarbonyl group contains 5 or 6 ring carbon atoms.

5. A nucleoside, nucleoside analog, nucleotide, nucleotide analog or oligonucleotide according to claim 4, wherein the cycloalkylcarbonyl group is cyclohexanecarbonyl.

6. A nucleoside, nucleoside analog, nucleotide, nucleotide analog or oligonucleotide according to claim 1, wherein the nucleobase B is a radical of formula I, Ia, Ib, Ic, Id or Ie 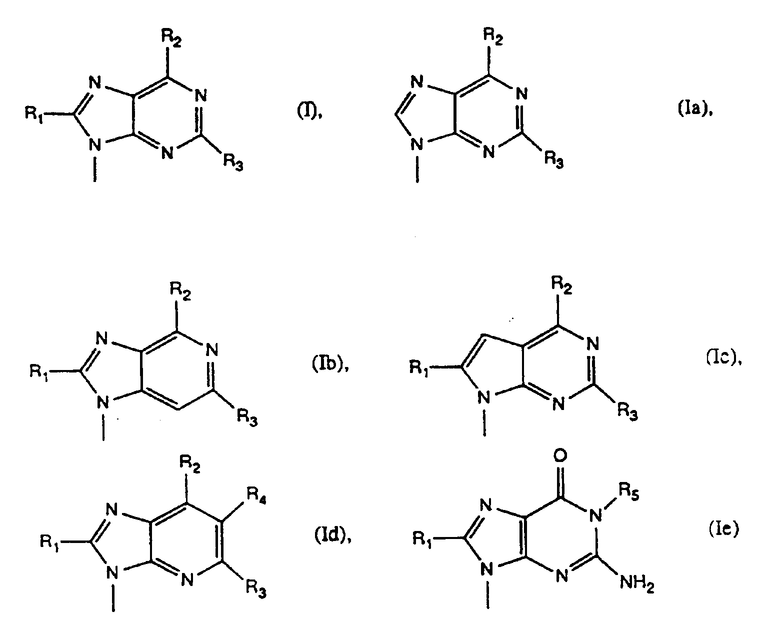 wherein R₁ is H, Cl, Br, NH₂ or OH, and R₂, R₃ and R₄ are each independently of one another H, OH, SH, NH₂, NHNH₂, NHOH, NHOalkyl containing 1 to 12 carbon atoms, F, Cl, Br, alkyl or hydroxyalkyl or aminoalkyl or alkoxy or alkylthio of 1 to 12 carbon atoms, where the hydroxyl and amino groups are unsubstituted or substituted by a protective group, or are phenyl, benzyl, primary amino containing 1 to 20 carbon atoms or secondary amino containing 2 to 30 carbon atoms, and R₅ is H or C₁-C₄alkyl.

7. A nucleoside, nucleoside analog, nucleotide, nucleotide analog or oligonucleotide according to claim 6, wherein the primary amino contains 1 to 12 carbon atoms and the secondary amino contains 2 to 12 carbon atoms.

8. A nucleoside, nucleoside analog, nucleotide, nucleotide analog or oligonucleotide according to claim 6, wherein the primary amino and secondary amino are radicals of formula R₆R₇N, wherein R₆ is H or independently has the meaning of R₇, and R₇ is C₁-C₂₀alkyl, C₁-C₂₀aminoalkyl, C₁-C₂₀hydroxyalkyl; carboxyalkyl or carbalkoxyalkyl in which the carbalkoxy moiety contains 2 to 8 carbon atoms and the alkyl moiety contains 1 to 6 carbon atoms; C₂-C₂₀alkenyl; phenyl, mono- or di(C₁-C₄alkyl- or -alkoxy)phenyl, benzyl, mono- or di(C₁-C₄alkyl- or -alkoxy)benzyl; or 1,2-, 1,3- or 1,4-imidazolyl-C₁-C₆alkyl, or R₆ and R₇, taken together, are tetra- or pentamethylene, 3-oxa-1,5-pentylene, -CH₂-NR₈-CH₂CH₂- or -CH₂CH₂-NR₈-CH₂CH₂-, wherein R₈ is H or C₁-C₄alkyl, and the amino group in the aminoalkyl is unsubstituted or substituted by one or two C₁-C₄alkyl or C₁-C₄hydroxyalkyl groups, and the hydroxyl group in the hydroxyalkyl is free or etherified with C₁-C₄alkyl.

9. A nucleoside, nucleoside analog, nucleotide, nucleotide analog or oligonucleotide according to claim 7, wherein the primary amino and secondary amino are methylamino, ethylamino, dimethylamino, diethylamino, allylamino, mono- or di(hydroxyeth-2-yl)amino, phenylamino, benzylamino, acetylamino, isobutyrylamino and benzoylamino.

10. A nucleoside, nucleoside analog, nucleotide, nucleotide analog or oligonucleotide according to claim 6, wherein R₁ in formulae I, Ib, Ic, Id and Ie is hydrogen.

11. A nucleoside, nucleoside analog, nucleotide, nucleotide analog or oligonucleotide according to claim 6, wherein R₄ in formula Id is hydrogen.

12. A nucleoside, nucleoside analog, nucleotide, nucleotide analog or oligonucleotide according to claim 6, wherein R₂ and R₃ in formulae I, Ia, Ib, Ic, Id and Ie are each independently of the other H, F, Cl, Br, OH, SH, NH₂, NHOH, NHNH₂, methylamino, dimethylamino, benzoylamino, methoxy, ethoxy and methylthio.

13. A nucleoside, nucleoside analog, nucleotide, nucleotide analog or oligonucleotide according to claim 6, wherein B is a purine radical or the radical of a purine analog from the series consisting of adenine, N-methyladenine, N-benzoyladenine, 2-methylthioadenine, 2-aminoadenine, 2-hydroxypurine, 2-amino-6-chloropurine, 2-amino-6-methylthiopurine, guanine and N-isobutyrylguanine.

14. A nucleoside, nucleoside analog, nucleotide, nucleotide analog or oligonucleotide according to claim 1, wherein the nucleobase B is a radical of formula II, IIa or IIb 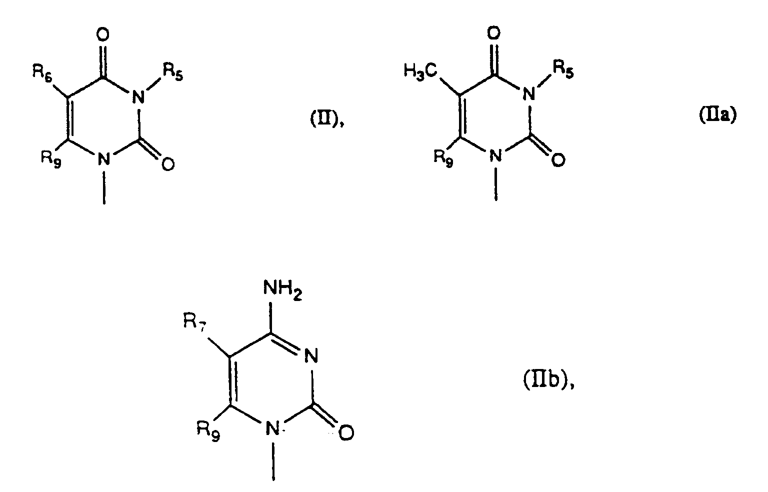 wherein R₅ is H or C₁-C₄alkyl and R₆, R₇ and R₉ each independently of one another are H, OH, SH, NH₂, NHNH₂, NHOH, NHOalkyl containing 1 to 12 carbon atoms, F, Cl, Br, alkyl or hydroxyalkyl or aminoalkyl or alkoxy or alkylthio containing 1 to 12 carbon atoms, where the hydroxyl and amino groups are unsubstituted or substituted by a protective group, or are phenyl, benzyl, primary amino containing 1 to 20 carbon atoms or secondary amino containing 2 to 30 carbon atoms, and the hydrogen atoms of the NH₂ group in formula IIb may be substituted by C₁-C₆alkyl or benzoyl, or a dihydro derivative thereof.

15. A nucleoside, nucleoside analog, nucleotide, nucleotide analog or oligonucleotide according to claim 14, wherein R₆ is H, C₁-C₆alkyl or C₁-C₆hydroxyalkyl, F, Cl, Br, NH₂, benzoylamino, mono- or di-C₁-C₆alkylamino.

16. A nucleoside, nucleoside analog, nucleotide, nucleotide analog or oligonucleotide according to claim 14, wherein R₇ is H, C₁-C₆alkyl or C₁-C₆alkoxy or C₁-C₆-hydroxyalkyl, F, Cl, Br, NH₂, benzoylamino, mono- or di-C₁-C₆alkylamino.

17. A nucleoside, nucleoside analog, nucleotide, nucleotide analog or oligonucleotide according to claim 16, wherein R₇ is H, F, Cl, Br, NH₂, NHCH₃, N(CH₃)₂ or C₁-C₄alkyl.

18. A nucleoside, nucleoside analog, nucleotide, nucleotide analog or oligonucleotide according to claim 16, wherein R₇ is H, C₁-C₄alkyl, NH₂, NHCH₃ or (CH₃)₂N.

19. A nucleoside, nucleoside analog, nucleotide, nucleotide analog or oligonucleotide according to claim 14, wherein B is a pyrimidine radical or a radical of a pyrimidine analog from the series consisting of uracil, thymine, cytosine, 5-fluorouracil, 5-chlorouracil, 5-bromouracil, dihydrouracil or 5-methylcytosine.

20. A nucleoside, nucleoside analog, nucleotide, nucleotide analog or oligonucleotide according to claim 1, which has one of the following one of the following formulae IIIa, IIIb, IIIc or IIId 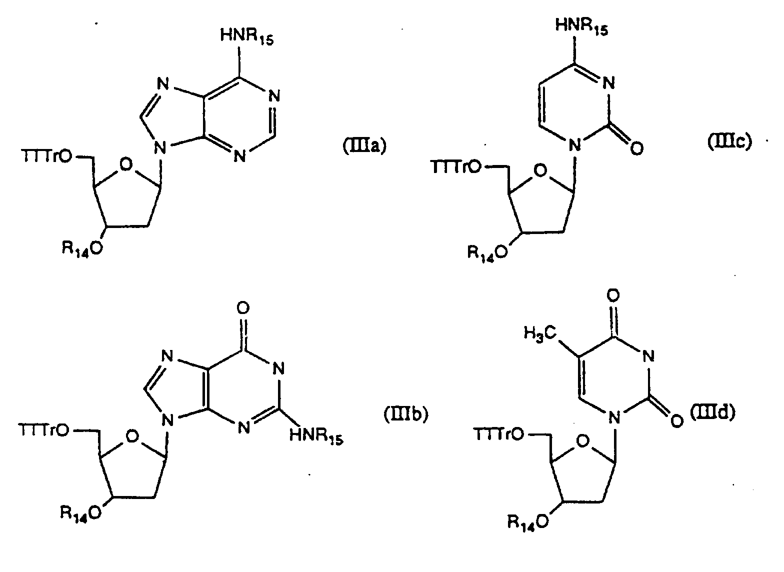 wherein R₁₄ is hydrogen or a radical which forms a nucleotide linking group and R₁₅ is hydrogen or cyclohexylcarbonyl.

21. A nucleoside, nucleoside analog, nucleotide, nucleotide analog or oligonucleotide according to claim 20, wherein R₁₄ is a phosphorus-containing radical of formula IVa, IVb or IVc 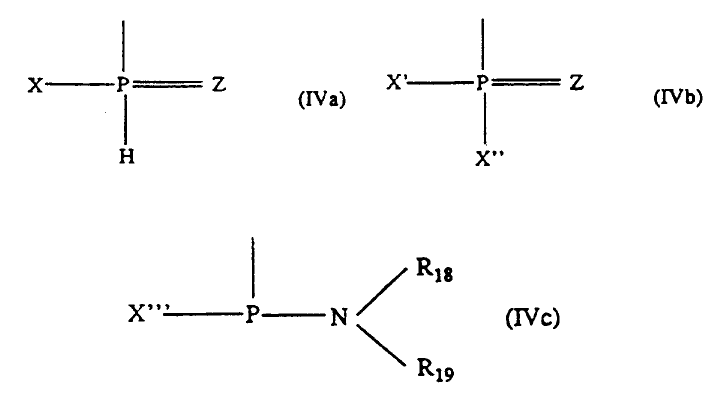 which forms a nucleotide linking group, wherein
Z is oxygen or sulfur;
X, X' and X'' are each independently of one another oxygen or sulfur carrying a negative charge, with counterion Li, Na, K, Cs, tertiary or quaternary ammonium; or X' and X'' are each independently of the other C₁-C₁₂alkyl, C₆-C₁₂-aryl, C₇-C₂₀aralkyl, C₇-C₂₀alkaryl, -OR_{b} or -SR_{b};
X''' is C₁-C₁₂alkyl, C₆-C₁₂aryl, C₇-C₂₀aralkyl, C₇-C₂₀-alkaryl, -OR_{b} or -SR_{b}; and
R_{b} is C₁-C₁₂alkyl, C₆-C₁₂aryl, C₇-C₂₀aralkyl, C₇-C₂₀alkaryl;
R₁₈ and R₁₉ are each independently of the other hydrogen, C₁-C₁₂alkyl, C₂-C₁₂alkenyl, C₆-C₁₂aryl, C₇-C₂₀aralkyl or C₇-C₂₀alkaryl;
and alkyl, aryl, aralkyl and alkaryl in R₁₈, R₁₉ and R_{b} are unsubstituted or substituted by alkoxy, alkylthio, halogen, -CN, -NO₂, phenyl, nitrophenyl or halophenyl.

22. A process for the preparation of a nucleoside or nucleoside analog according to claim 1, which comprises reacting a nucleoside or nucleoside analog with tris4,4',4''-tert-butyltrityl chloride in the presence of a sterically hindered tertiary amine in a suitable aprotic, polar, basic solvent.

23. A process for the preparation of an oligonucleotide of formula V
5'OH(U)ₘ(V)ₙOH-3' (V)
wherein U and V are identical or different, natural or synthetic nucleoside residues and m and n are each independently of the other 0 or a number from 1 to 200, and the sum of m and n is 2 to 200, by
(a) reacting a compound of formula VI
R₂₀-0-(U')_{m'}(V')_{n'}OR₂₁-3' (V)
wherein R₂₀ is a protective group and U' and V' have the meanings of U and V, m' and n' are each independently of the other 0 or a number from 1 to 199, and the sum of m' and n' is 2 to 199, and R₂₁ is a phosphorus-containing radical of formula IVa, IVb or IVc 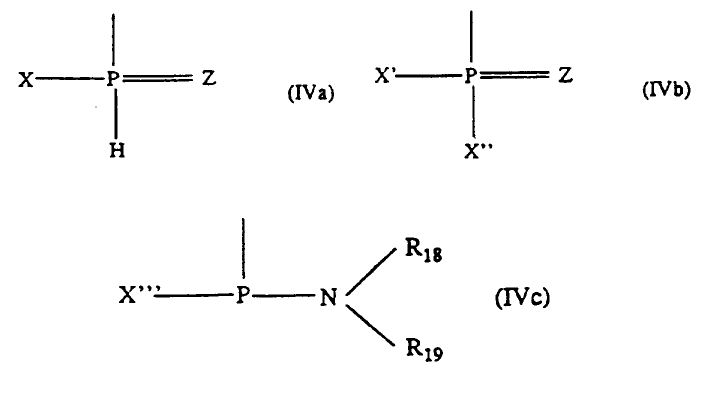 which forms a nucleotide linking group, wherein
Z is oxygen or sulfur;
X, X' and X'' are each independently of one another oxygen or sulfur carrying a negative charge, with counterion Li, Na, K, Cs, tertiary or quaternary ammonium; or
X' and X'' are each independently of the other C₁-C₁₂alkyl, C₆-C₁₂aryl, C₇-C₂₀aralkyl, C₇-C₂₀alkaryl, -OR_{b} or -SR_{b};
X''' is C₁-C₁₂alkyl, C₆-C₁₂aryl, C₇-C₂₀aralkyl, C₇-C₂₀-alkaryl, -OR_{b} or -SR_{b}; and
R_{b} is C₁-C₁₂alkyl, C₆-C₁₂aryl, C₇-C₂₀aralkyl, C₇-C₂₀alkaryl;
R₁₈ and R₁₉ are each independently of the other hydrogen, C₁-C₁₂alkyl, C₂-C₁₂alkenyl, C₆-C₁₂aryl, C₇-C₂₀aralkyl or C₇-C₂₀alkaryl;
and alkyl, aryl, aralkyl and alkaryl in R₁₈, R₁₉ and R_{b} are unsubstituted or substituted by alkoxy, alkylthio, halogen, -CN, -NO₂, phenyl, nitrophenyl or halophenyl, with a compound of formula VII
5'-OH(U'')_{m''}(V'')_{n''}O-R₂₂ (VII)
wherein U'' and V'' have the meanings of U and V, m'' and n'' are each independently of the other 0 or a number from 1 to 198, and the sum of m'' and n'' is 2 to 198, and R₂₂ is
(i) a radical of formula IVb, wherein Z is oxygen or sulfur; X' and X'' are each independently of the other C₁-C₁₂alkyl, C₆-C₁₂aryl, C₇-C₂₀aralkyl, C₇-C₂₀alkaryl, -OR_{b} or -SR_{b}; and R_{b} is C₁-C₁₂alkyl, C₆-C₁₂aryl, C₇-C₂₀aralkyl, C₇-C₂₀alkaryl, and alkyl, aryl, aralkyl and alkaryl in R_{b} are unsubstituted or substituted by alkoxy, alkylthio, halogen, -CN, -NO₂, phenyl, nitrophenyl or halophenyl;
(ii) a hydroxyl protective group; or
(iii) a linkage to a solid carrier material by means of a linker;
(b) if desired, repeating step (a) until an oligonucleotide of the desired length has formed, and, before each coupling, removing the protective group R₂₀, capping any free hydroxyl groups present, and then oxidizing the resultant phosphite to the phosphate,
(c) if desired, detaching or isolating the oligonucleotide, and
(d) removing the residual protective group R₂₀, wherein the protective group R₂₀ is TTTr.

24. A process according to claim 23, wherein the sum of m and n is 2 to 50.

25. A process acording to claim 24, wherein the sum of m and n is 2 to 30.

26. Use of a nucleotide, nucleotide analog or oligonucleotide according to claim 1 for the preparation of an oligonucleotide of formula V
5'-OH(U)ₘ(V)ₙOH-3' (V)
wherein U and V are identical or different, natural or synthetic nucleoside residues and m and n are each independently of the other 0 or a number from 1 to 200, and the sum of m and n is 2 to 200.

27. A compound according to claim 2, wherein the amidine protective group is dimethylaminomethylidene.

28. A nucleoside, nucleoside analog, nucleotide, nucleotide analog or oligonucleotide according to claim 1, wherein the primary protected hydroxyl group is at position 5' of the parent structure.

29. Use of the radical tris-4,4',4''-tert-butyltrityl as protective group for a primary hydroxyl group of the parent structure of a nucleoside, nucleoside analog, nucleotide, nucleotide analog or oligonucleotide comprising at least two such identical or different nucleotides and/or nucleotide analogs, whose parent structure comprises an unsubstituted or substituted residue of a nucleobase B and one primary hydroxyl group.

30. Use according to claim 29, wherein the primary hydroxyl group is at position 5' of the parent structure.

## Revendications

1. Un nucléoside, un analogue de nucléoside, un nucléotide, un analogue de nucléotide ou un oligonucléotide constitué d'au moins deux de ces nucléotides et/ou analogues de nucléotides identiques ou différents dont le squelette de base contient un reste éventuellement substitué d'une base nucléique B et un groupe hydroxyle primaire protégé, caractérisé en ce que le groupe protecteur est le tris-4, 4', 4''-tert-butyltrityle.

2. Un nucléoside, un analogue de nucléoside, un nucléotide, un analogue de nucléotide ou un oligonucléotide selon la revendication 1, caractérisé en ce qu'il est protégé en plus par un groupe cycloalkylcarbonyle comportant 3 à 12 atomes de carbone cyclique, non substitué ou substitué par un alkyle en C₁-C₆ ou par un groupe protecteur de l'amidine.

3. Un nucléoside, un analogue de nucléoside, un nucléotide, un analogue de nucléotide ou un oligonucléotide selon la revendication 2, caractérisé en ce que le groupe cycloalkylcarbonyle contient 4 à 8 atomes de carbone cycliques.

4. Un nucléoside, un analogue de nucléoside, un nucléotide, un nucléotide, un analogue de nucléotide ou un oligonucléotide selon la revendication 3, caractérisé en ce que le groupe cycloalkylcarbonyle contient 5 ou 6 atomes de carbone cycliques.

5. Un nucléoside, un anlogue de nucléoside, un nucléotide, un analogue de nucléotide ou un oligonucléotide selon la revendication 4, caractérisé en ce que le groupe cycloalkylcarbonyle est le cyclohexanecarbonyle.

6. Un nucléoside, un analogue de nucléoside, un nucléotide, un analogue de nucléotide ou un oligonucléotide selon la revendication 1, où la base nucléique B est un reste de formule I, Ia, Ib, Ic , Id ou Ie 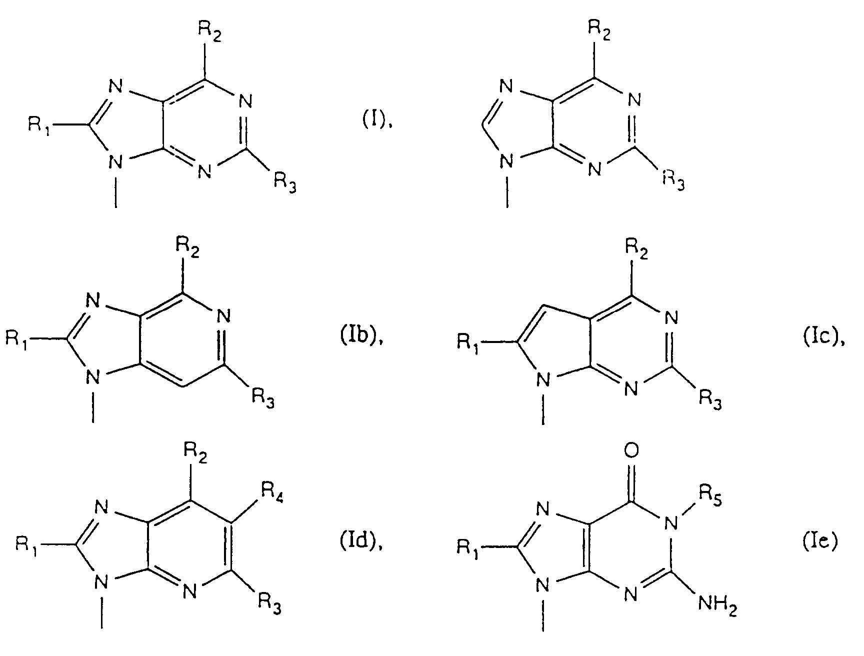 où R₁ représente H, Cl, Br, NH₂ ou OH et R₂, R₃ et R₄ représentent, indépendamment les uns des autres, H, OH, SH, NH₂, NHNH₂, NHOH, NHOalkyle comportant 1 à 12 atomes de C, F, Cl, Br, un alkyle ou un hydroxyalkyle ou un aminoalkyle ou un alcoxy ou un alkylthio comportant 1 à 12 atomes de C, les groupes hydroxyles et amino étant non substitués ou substitués par un groupe protecteur, le phényle, le benzyle, un amino primaire comportant 1 à 20 atomes de C ou un amino secondaire comportant 2 à 30 atomes de C et R₅ représente H ou un alkyle en C₁-C₄.

7. Un nucléoside, un analogue de nucléoside, un nucléotide, un analogue de nucléotide ou un oligonucléotide selon la revendication 6 où l'amino primaire contient 1 à 12 atomes de C et l'amino secondaire contient 2 à 12 atomes de C.

8. Un nucléoside, un analogue de nucléoside, un nucléotide, un analogue de nucléotide ou un oligonucléotide selon la revendication 6 où l'amino primaire et l'amino secondaire sont des restes de formule R₆R₇N dans laquelle R₆ représente U ou a la signification de R₇ et R₇ représente un alkyle, ou un aminoalkyle ou un hydroxyalkyle en C₁-C₂₀ ; un carboxyalkyle ou un carbalcoxyalkyle, le groupe carbalcoxy contenant 2 à 8 atomes de C et le groupe alkyle 1 à 6 atomes de C ; un alcényle en C₂-C₂₀) ; le phényle, un mono- ou di-(alkyle ou alcoxy en C₁-C₄)-phényle, le benzyle, un mono- ou di-(alkyle ou alcoxy en C₁-C₄)-benzyle ; ou le 1,2-, 1,3- ou 1,4-imidazolylalkyle en C₁-C₆ ou R₆ et R₇ représentent ensemble le tétra- ou pentaméthylène, le 3-oxa-1,5-pentylène, -CH₂-NR₈-CH₂-CH₂- ou CH₂CH₂-NR₈-CH₂CH₂ ou R₈ représente H ou un alkyle en C₁-C₄, le groupe amino dans l'aminoalkyle étant non substitué ou substitué par un ou deux groupes alkyles ou hydroxyalkyles en C₁-C₄ et le groupe hydroxyle dans l'hydroxyalkyle étant éventuellement éthérifié par un alkyle en C₁-C₄.

9. Un nucléoside, un analogue de nucléoside, un nucléotide, un analogue de nucléotide ou un oligonucléotide selon la revendication 7 où l'amino primaire et l'amino secondaire sont le méthylamino, l'éthylamino, le diméthylamino, le diéthylamino, l'allylamino, le mono- ou di-(hydroxy-éth-2-yl)-amino, le phénylamino, le benzylamino, l'acétylamino et le benzoylamino.

10. Un nucléoside, un analogue de nucléoside, un nucléotide, un analogue de nucléotide ou un oligonucléotide selon la revendication 6, où R₁ dans les formules I, Ib, Ic, Id et Ie représente l'hydrogène.

11. Un nucléoside, un analogue de nucléoside, un nucléotide, un analogue de nucléotide ou un oligonucléotide selon la revendication 6, où R₄ dans la formule Id représente l'hydrogène.

12. Un nucléoside, un analogue de nucléoside, un nucléotide, un analogue de nucléotide ou un oligonucléotide selon la revendication 6, où R₂ et R₃ dans les ftomules I, Ia, Ib, Ic, Id et Ie représentent, indépendamment l'un de l'autre, H, F, Cl, Br, OH, SH, NH₂, NHOH, NHNH₂, le méthylamino, le diméthylamino, le benzoylamino, le méthoxy, l'éthoxy et le méthylthio.

13. Un nucléoside, un analogue de nucléoside, un nucléotide, un analogue de nucléotide ou un oligonucléotide selon la revendication 6 où B est un reste de la purine ou un reste d'un analogue de la purine provenant du groupe constitué par l'adénine, la N-méthyladénine, la N-benzoyladénine, la 2-méthylthioadénine, la 2-aminoadénine, la 2-hydroxypurine, la 2-amino-6-chloropurine, la 1-amino-6-méthylthiopurine, la guanine et la N-isobutyrylguanine.

14. Un nucléoside, un analogue de nucléoside, un nucléotide, un analogue de nucléotide ou un oligonucléotide selon la revendication 1, où la base nucléique B est un reste de formules II, IIa ou IIb 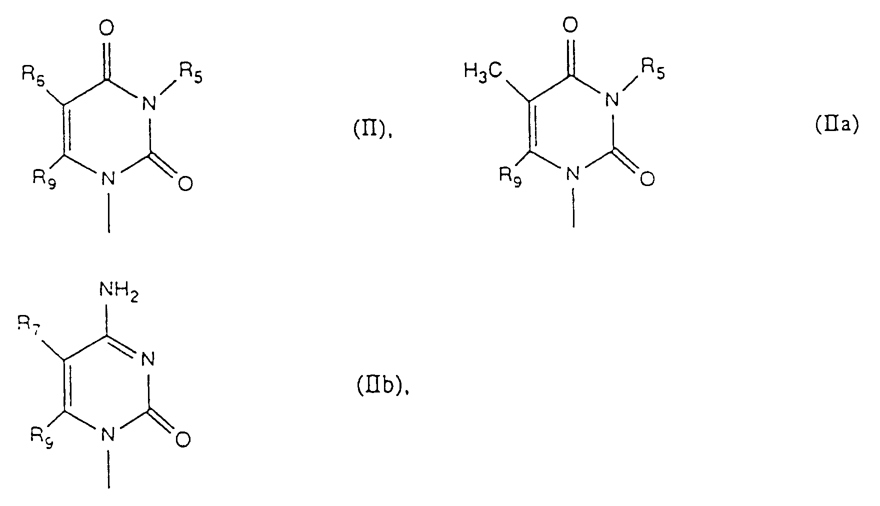 où R₅ renrésente H, ou un alkyle en C₁-C₄, et R₆, R₇ et R₈ représentent, indépendamment les uns des autres, H, OH, SH, NH₂, NHNH₂, NHOH, NHOalkyle comportant 1 à 12 atomes de C, F, Cl, Br, un alkyle ou un hydroxyalkyle ou un aminoalkyle ou un alcoxy ou un alkylthio comportant 1 à 12 atomes de C, les groupes hydroxyles et amino étant non substitués ou substitués par un groupe protecteur, le phényle, le benzyle, un amino primaire comportant 1 à 20 atomes de C ou un amino secondaire comportant 2 à 30 atomes de C et le 9 atomes d'hydrogène du groupe NH₂ dans la formule IIb pouvant être substitués par un alkyle en C₁-C₆ ou le benzoyle, ainsi que leurs dérivés dihydro.

15. Un nucléoside, un analogue de nucléoside, un nucléotide, un analogue de nucléotide ou un oligonucléotide selon la revendication 14, où R₆ représente H, alkyle ou un hydroxyalkyle en C₁-C₆, F, Cl, Br, NH₂, le benzoylamino, un mono- ou dialkyle en C₁-C₆ amino.

16. Un nucléoside, un analogue de nuléoside, un nuléotide, un analogue de nucléotide ou un oligonucléotide selon la revendication 14, où R₇ représente H, un alkyle ou un alcoxy ou un hydroxyalkyle en C₁-C₆, F, Cl, Br, NH₂, le benzoylamino, un mono- ou dialkyle en C₁-C₆-amino.

17. Un nucléoside, un analogue de nucléoside, un nucléotide, un analogue de nucléotide ou un oligonucléotide selon la revendication 16, où R₇ représente H, F, Cl, Br, NH₂, NHCH₃, N(CH₃)₂ ou un alkyle en C₁-C₄.

18. Un nucléoside, un analogue de nucléoside, un nucléotide, un analogue de nucléotide ou un oligonucléotide selon la revendication 16, où R₇ représente H, un alkyle en C₁-C₄, NH₂, NHCH₃ ou (CH₃)₂N.

19. Un nucléoside, un analogue de nucléoside, un nucléotide, un analogue de nucléotide ou un oligonucléotide selon la revendication 14, où B est un reste de la pyrimidine ou un reste d'un analogue de la pyrimidine provenant du groupe constitué par l'uracile, le thymine, la cytosine, le 5-fluorouracile, le 5-chlorouracile, le 5-bromouracile, le dihydrouracile ou la 5-méthylcytosine.

20. Un nucléoside, un analogue de nucléoside, un nuclélotide, un analogue de nucléotide ou un oligonucléotide selon la revendication 1, caractérisé en ce qu'il présente l'une des formules IIIa, IIIb, IIIc ou IIId suivantes 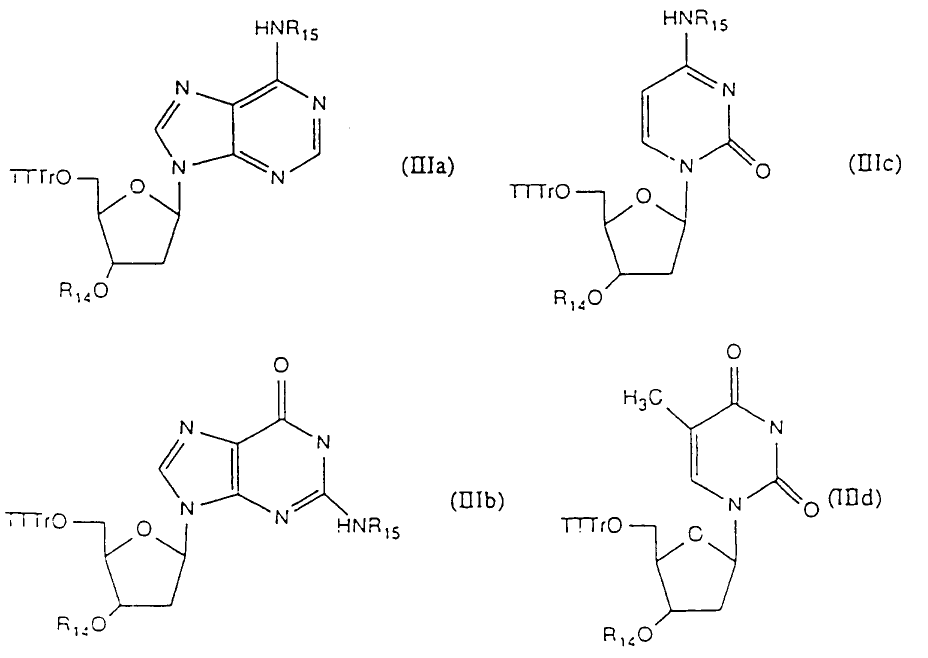 où R₁₄ représente l'hydrogène ou un reste formant un groupe pontal pour les nucléotides et R₁₅ représente l'hydrogène ou le cyclohexylcarbonyle.

21. Un nucléoside, un analogue de nucléoside, un nucléotide, un analogue (le nucléotide ou un oligonucléotide selon la revendication 20, caractérisé en ce que R₁₄ représente un reste de formules IVa, IVb ou IVc, phosphoré, formant un groupe pontal pour les nucléotides 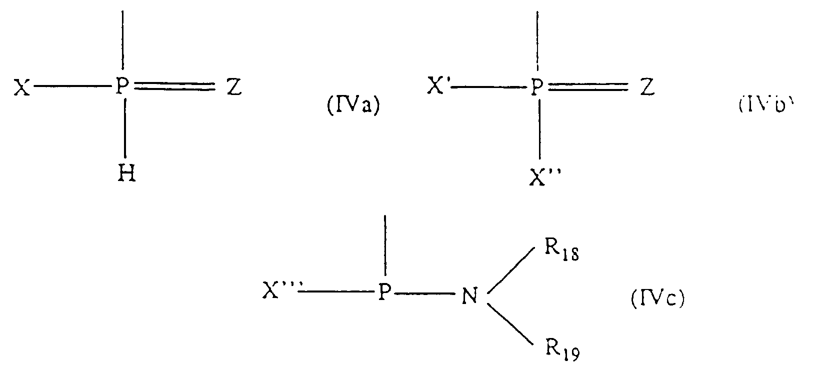 où
Z représente l'oxygène ou le soufre ;
X, X' et X'' représentent, indépendamment les uns des autres, l'oxygène ou le soufre ayant une charge négative, avec comme ions complémentaires Li, Na, K, Cs, ou un ammonium tertiaire ou quaternaire ; ou
X' et X'' représentent, indépendamment l'un de l'autre, un alkyle en C₁-C₁₂, un aryle en C₆-C₁₂, un aralkyle en C₇-C₂₀, un alkaryle en C₇-C₂₀, -OR_{b} ou -SR_{b} ;
X''' représente un alkyle en C₁-C₁₂, un aryle en C₆-C₁₂, un aralkyle en C₇-C₂₀, un alkaryle en C₇-C₂₀, -OR_{b}, ou -SR_{b}; et
R_{b} représente un alkyle en C₁-C₁₂, un aryle en C₆-C₁₂, un aralkyle en C₇-C₂₀, un alkaryle en C₇-C₂₀ ;
R₁₈ et R₁₉ représentent, indépendamment l'un de l'autre, l'hydrogène, un alkyle en C₁-C₁₂, un alcényle en C₂-C₁₂, un aryle en C₆-C₁₂, un aralkyle en C₇-C₂₀ ou un alkaryle en C₇-C₂₀ ;
les alkyles, aryles, aralkyles et alkaryles dans R₁₈, R₁₉ et R_{b} étant non substitués ou substitués par un alcoxy, un alkylthio, un halogène, -CN, -NO₂, le phényle, le nitrophényle ou un halogénophényle.

22. Procédé pour la préparation des nucléosides et analogues de nucléosides selon la revendication 1, caractérisé en ce que l'on fait réagir un nucléoside ou un analogue de nucléoside avec le chlorure de tris-4,4',4''-tert-butyltrityle en présence d'une amine tertiaire stériquement empêchée dans un solvant basique, aprotique, polaire, approprié.

23. Un procédé pour la préparation des oligonucléotides de formule V
5'-OH(U)ₘ(V)ₙOH-3' (V)
où U et V représentent des restes nucléosides, naturels ou synthétiques, identiques ou différents, et m et n sont, indépendamment l'un de l'autre, égal à 0 ou un nombre compris entre 1 et 200, la somme de m et n étant comprise entre 2 et 200, dans lequel
(a) on fait réagir un composé de formule VI
X₂₀-O-(U')_{m'}(V')_{n'}OR₂₁-3' (VI)
où R₂₀ est un groupe protecteur et U' et V' ont les significations de U et V, m' et n' sont, indépendamment l'un de l'autre, égal à 0 ou un nombre compris entre 1 et 199, la somme de m' et n' étant comprise entre 2 et 199 et R₂₁ représente un reste de formules IVa, IVb ou IVc, phosphoré, formant un groupe pontal pour les nucléotides 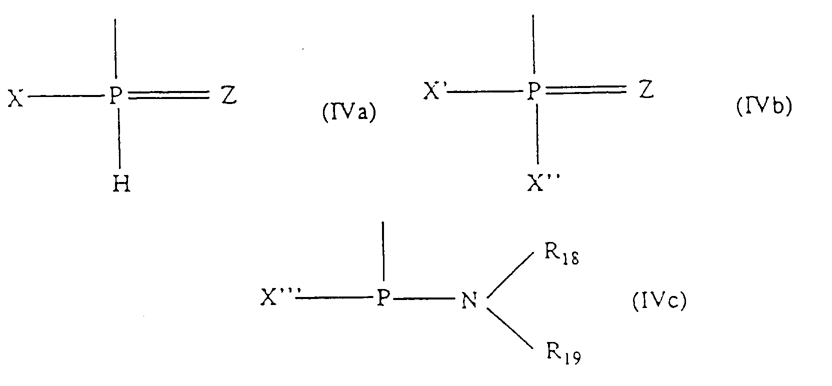 où
Z représente l'oxygène ou le soufre ;
X, X' et X'' représentent, indépendamment les uns des autres, l'oxygène ou le soufre ayant une charge négative, avec comme ions complémentaires Li, Na, K, Cs, un ammonium tertiaire ou quaternaire ; ou
X' et X'' représentent, indépendamment l'un de l'autre, un alkyle en C₁-C₁₂, un aryle en C₆-C₁₂, un aralkyle en C₇-C₂₀, un alkaryle en C₇-C₂₀, -OR_{b} ou -SR_{b} ;
X''' représente un alkyle en C₁-C₁₂, un aryle en C₆-C₁₂, un aralkyle en C₇-C₂₀, un alkaryle en C₇-C₂₀, -OR_{b} ou -SR_{b} ; et
X_{b} représente un alkyle en C₁-C₁₂, un aryle en C₆-C₁₂, un aralkyle en C₇-C₂₀, un alkaryle en C₇-C₂₀ ;
X₁₈ et R₁₉ représentent, indépendamment l'un de l'autre, l'hydrogène, un alkyle en C₁-C₁₂, un alcényle en C₂-C₁₂, un aryle en C₆-C₁₂, un aralkyle en C₇-C₂₀ ou un alkaryle en C₇-C₂₀ ;
les alkyles, aryles, aralkyles et alkaryles dans R₁₈, R₁₉ et R_{b} étant non substitués ou substitués par un alcoxy, un alkylthio, un halogène, -CN, -NO₂, le phényle, le nitrophényle ou un halogénophényle,
avec un composé de formule VII
5'-OH(U'')_{m''}(V'')_{n''}O-R₂₂ (VII)
où U'' et V'' ont les significations de U et V, m'' et n'' sont, indépendamment l'un de l'autre, égal à 0 ou un nombre compris entre 1 et 198, la somme de m'' et n'' étant comprise entre 2 et 198, et R₂₂ représente
(i) un reste de formule IVb où Z représente l'oxygène ou le soufre ; X' et X'' représentent, indépendamment l'un de l'autre, un alkyle en C₁-C₁₂, un aryle en C₆-C₁₂, un aralkyle en C₇-C₂₀, un alkaryle en C₇-C₂₀, -OR_{b} ou -SR_{b} ; et R_{b} représente un alkyle en C₁-C₁₂, un aryle en C₆-C₁₂, un aralkyle en C₇-C₂₀, un alkaryle en C₇-C₂₀, les alkyles, aryles, aralkyles et alkaryles dans R_{b} étant non substitués ou substitués par un alcoxy, un alkylthio, un halogène, -CN, -NO₂, le phényle, le nitro-phényle ou un halogénophényle;
(ii) un groupe protecteur de l'hydroxy; ou
(iii) une liaison à un support solide à l'aide d'un "linker";
(b) on répète éventuellement l'étape (a) jusqu'à l'obtention d'un oligonucléotide de la longueur désirée, le groupe protecteur R₂₀ étant éliminé avant chaque nouveau couplage, les groupes hydroxyles libres éventuellement présents étant bloqués (capping), puis le phosphite formé étant oxydé en phosphate,
(c) on libère éventuellement l'oligonucléotide ou à l'isoler et
(d) on sépare le groupe protecteur R₂₀ restant,
caractérisé en ce que le groupe protecteur R₂₀ est le TTTr.

24. Un procédé selon la revendication 23, caractérisé en ce que la somme de m et n est un nombre compris entre 2 et 50.

25. Un procédé selon la revendication 24, caractérisé en ce que la somme de m et n est un nombre compris entre 2 et 30.

26. Utilisation d'un nucléotide, d'un analogue de nucléotide, on d'un oligonucléotide selon la revendication 1 pour la préparation des oligonucléotides de formule V
5'-OH(U)ₘ(V)ₙOH-3' (V)
où U et V représentent des restes nucléosides, naturels ou synthétiques, identiques ou différents, et m et n sont, indépendamment l'un de l'autre, égal à 0 ou un nombre compris entre 1 et 200, la somme de m et n étant comprise entre 2 et 200.

27. Un composé selon la revendication 2, caractérisé en ce que le groupe protecteur de l'amidine est le diméthylaminométhylidène.

28. Un nucléoside, un analogue de nucléoside, un nucléotide, un analogue de nucléotide ou un oligonucléotide selon la revendication 1, caractérisé en ce que le groupe hydroxyle primaire protégé se trouve en position 5' sur le squelette de base.

29. Utilisation du reste tris-4,4',4''-tert-butyltrityle comme groupe protecteur d'un groupe hydroxyle primaire du squelette de base des nucléosides, des analogues de nucléosides, des nucléotides, des analogues de nucléotides ou des oligonucléotides constitués d'au moins deux de ces nucléotides et/ou analogues de nucléotides identiques ou différents dont le squelette de base contient un reste éventuellement sabstitué d'une base nucléique B et un groupe hydroxyle primaire.

30. Utilisation selon la revendication 29, caractérisée en ce que le groupe hydroxyle primaire se trouve en position 5 sur le squelette de base.
